# EUROPEAN PATENT APPLICATION

(11) **EP 4 563 284 A2**
(43) Date of publication of application: **04.06.2025**
(21) Application number: 24183092.6
(22) Date of filing: 19.06.2024
(51) Int. Cl.: B25B 13/46, B25B 13/48, B25B 17/00, B25B 23/00, B25B 23/14, B25B 23/142, B25B 21/00, A61B 17/70, A61B 17/88, A61B 90/00

(54) **COUNTER-TORQUE DRIVER TOOL**

(30) Priority: 19.07.2023 US 202318355077
(71) Applicant: C-Torq, Inc., Moreland Hills Ohio 44022 (US)
(72) Inventor: NILSSON, Carl Michael, Moreland Hills Ohio 44022 (US)
(74) Representative: IK-IP LTD

(57) **Abstract**

One or more techniques and/or systems are disclosed for a tool driver device that can apply counter-torque to the device while applying torque to a target component that is engaging a target base. The example device can be operated by one hand to actuate a power input component that provides the desired torque, while providing counter-torque to offset rotation of the device. A selectably engaged counter-torque component is selected to complement the target base, to operably hold it in place during use. A power input provides power to apply rotational force to drive a selectably coupled tool, which has been selected to complement the target component engaged with a target base. The counter-torque component is operably engaged with the base, which applies counter-torque to the torque provided by the target component.

## Description

### CROSS-REFERENCE TO RELATED APPLICATIONS

This application is a continuation-in-part (CIP) of U.S. Patent Application Serial No. 17/898,900, filed on August 30, 2022, which claims priority to U.S. Provisional Patent Application Serial No. 63/264,117, entitled COUNTER-TORQUE FASTENER DRIVER, filed on November 16, 2021, and claims priority to U.S. Provisional Patent Application Serial No. 63/238,522, entitled COUNTER-TORQUE FASTENER DRIVER, filed on August 30, 2021, each of which is incorporated herein by reference in its entirety.

### BACKGROUND

A driver-type tool can be used to drive a fastener into (or out of) a target component. When driving the fastener into the target component torque is applied to the fastener by the driver, and counter-torque can be applied to the driver by a user (e.g., manually). When torque is applied to the fastener counter-torque is applied to the driver and/or the target component in order to allow the fastener to drive into the component (e.g., due to friction, etc.), otherwise either the target component will rotate with the fastener, or the driver will rotate in the intended rotational direction of the shaft. Typically, the needed counter-torque must be applied by the user with a separate device and/or some type of external stabilizing frame.

### SUMMARY

This Summary is provided to introduce a selection of concepts in a simplified form that are further described below in the Detailed Description. This Summary is not intended to identify key factors or essential features of the claimed subject matter, nor is it intended to be used to limit the scope of the claimed subject matter.

One or more techniques and systems are described herein for a tool driver device that applies torque to a target tool, such as one that is configured to engage a target component, while applying counter-torque to a target base that is engaged with the target component. That is, for example, the exemplary device can apply torque to a tool, such as a screwdriver head or socket head, etc., to drive a complementary target component, such as a screw, nut or bolt, etc. In this example, counter-torque can be applied to target base with which the target component is engaged, such as a surface, a frame, nut or bolt, etc., to counter- rotation of the target base. In this way, for example, the counter-rotation may be transferred to the device itself, making for easier operation. In some implementations, the exemplary device can provide for a one-hand operation, utilizing a type of trigger that, when activated, applies a desired amount of torque at the target tool end, while mitigating counter-rotation of the device in the user's hand.

In one implementation, the exemplary device applies torque to rotate a target tool engaging a complementary target component and applies counter torque to stabilize the device with regard to a target base engaged with the target component. In this implementation, the exemplary device comprises a housing that is configured to be operably held by an operator. Further, the exemplary device comprises a rotating shaft that rotates relative to the housing to operably provide torque to the target tool. A power input operably provides power to the rotating shaft. Additionally, a counter-torque component comprises a body that is configured to selectably, fixedly engage with the target base to apply counter-torque to the target base.

To the accomplishment of the foregoing and related ends, the following description and annexed drawings set forth certain illustrative aspects and implementations. These are indicative of but a few of the various ways in which one or more aspects may be employed. Other aspects, advantages and novel features of the disclosure will become apparent from the following detailed description when considered in conjunction with the annexed drawings.

### BRIEF DESCRIPTION OF THE DRAWINGS

FIGURES 1A through 1G are component diagram illustrating one example implementation of a fastener driving device that can be operated one-handed and can provide torque and counter-torque.
FIGURES 2A through 2G are component diagram illustrating another example implementation of a fastener driving device that can be operated one-handed and can provide torque and counter-torque.
FIGURES 3A and 3B are component diagram illustrating two example implementations of alternate means for activating the tool driver device.
FIGURES 4A through 4D are component diagram illustrating example implementations of one or more portions of the one or more systems and devices described herein.
FIGURES 5A and 5B are component diagram illustrating an alternate example implementation of one or more portions of the one or more systems and devices described herein.
FIGURE 6 is a component diagram illustrating a cut-away view of one exemplary implementation of a fastener driving device that can be operated one-handed and can provide torque and counter-torque.
FIGURES 7A through 7C are component diagram illustrating example implementations of one or more portions of the one or more systems and devices described herein, where a direction of the applied torque can be controlled.
FIGURES 8A through 8H are component diagram illustrating example implementations of one or more portions of the one or more systems and devices described herein, where an amount of torque applied can be controlled.
FIGURE 9 is a component diagram illustrating an example implementation of one or more portions of systems and devices described herein, such as a torque limiter component.
FIGURE 10 is a component diagram illustrating an example implementation of one or more portions of systems and devices described herein, such as a ratcheting directional control.
FIGURES 11A, 11B, 11C, and 11D are component diagrams illustrating an example implementation of one or more portions of systems and devices described herein, such as a component to change rotational direction of the output shaft.
FIGURE 12 is a component diagram illustrating an example implementation of one or more portions of systems and devices described herein, such as a driver head holder.
FIGURE 13 is a component diagram illustrating an example implementation of one or more portions of systems and devices described herein, such as another driver head holder.
FIGURE 14 is a component diagram illustrating an example where one or more portions of systems and devices described herein may be implemented, such as to drive a threaded fastener.
FIGURES 15A and 15B are component diagram illustrating one example implementation of one or more portions of the one or more systems and devices described herein.
FIGURES 16A and 16B are component diagram illustrating another example implementation of one or more portions of the one or more systems and devices described herein.
FIGURES 17A, 17B, and 17C are component diagram illustrating an example implementation of one or more portions of the one or more systems described herein, such as a torque limiter with variable values of torque.
FIGURES 18A, 18B, 18C, 18D, 18E, 18F, and 18G are component diagram illustrating an example implementation of one or more portions of the one of more systems described herein, such as torque limiter spring.
FIGURES 19A, 19B, 19C, 19D, 19E, 19F, 19G, and 19H are component diagram illustrating an example implementation of one or more portions of the one of more systems described herein, such as an angled driver and/or a stationary driver shaft with a rotational outer tube.
FIGURES 20A, 20B, and 20C are component diagram illustrating an example implementation of one or more portions of the one of more systems described herein, such as a driver adapted for non-manual power.
FIGURES 21A, 21B, 21C, and 21D are component diagram illustrating an example implementation of one or more portions of the one of more systems described herein, such as a nut-bolt driver.
FIGURES 22A and 22B are flowcharts illustrating an example implementation of combinations of one or more portions of the one of more systems described herein.
FIGURE 23 is a component diagram, in perspective view, illustrating an example implementation of one or more portions of one or more systems and devices described herein, such as a torque limiter module.
FIGURE 24 is a component diagram, in exploded view, illustrating an example implementation of one or more portions of one or more systems and devices described herein, such as the torque limiter module of FIGURE 24.
FIGURE 25 is a component diagram, in perspective view, illustrating an example implementation of one or more portions of one or more systems described herein.
FIGURE 26 is a component diagram, in perspective view, illustrating an example implementation of one or more portions of one or more systems described herein.
FIGURE 27 is a component diagram, in plan view, illustrating an example implementation of one or more portions of one or more systems and devices described herein, such as the torque limiter module of FIGURES 23 and 24.
FIGURES 28A, 28B, 28C, and 28D are component diagrams illustrating an example implementation of one or more portions of one or more systems and devices described herein, such as a torque limiter module during torque transfer.
FIGURE 29 is a component diagram illustrating an example implementation of one or more portions of one or more systems and devices described herein, such as a torque limiter module during tightening.
FIGURE 30 is a component diagram illustrating an example implementation of one or more portions of one or more systems and devices described herein, such as a torque limiter module during untightening.
FIGURE 31 is a component diagram, in exploded view, illustrating the example implementation of the device that is power driven for applying torque and counter-torque.
FIGURE 32 is a component diagram illustrating one example implementation of a device that is power driven for applying torque and counter-torque.
FIGURE 33 is a schematic diagram illustrating one example implementation of a device that is power driven for applying torque and counter-torque.

### DETAILED DESCRIPTION

The claimed subject matter is now described with reference to the drawings, wherein like reference numerals are generally used to refer to like elements throughout. In the following description, for purposes of explanation, numerous specific details are set forth in order to provide a thorough understanding of the claimed subject matter. It may be evident, however, that the claimed subject matter may be practiced without these specific details. In other instances, structures and devices are shown in block diagram form in order to facilitate describing the claimed subject matter.

In one aspect, a device that applies torque to a tool (e.g., fastener tool, drill, other rotating tools) disposed at the end of a rotating shaft can be used to drive a target component (e.g., fastener, auger, etc.) into a target base (e.g., surface, earth, other targeted parts). Further, in this aspect, a counter-torque component can be fixedly engaged with the housing and/or handle of the device, where the counter-torque component is designed to engage with the target base. In this way, for example, the base can be stabilized (e.g., have sufficient mass, and/or be fastened/fixed to a stabilizing mass) to mitigate rotation of the base with the target component to which it is engaged. In this example, the stabilization of the base can provide a counter-torque that is transferred to the device housing by way of the counter-torque component.

As one example, in this aspect, the exemplary device may allow a surgeon to use merely one hand to place a screw (e.g., tighten a set screw) in a spinal stabilization device, while it drives (e.g., applies the torque to) the set screw while also holding on to the spinal stabilizer or spinal rod to apply a counter torque to the device in place. As another example, the driver can be used to tighten a nut and bolt construct (e.g., or loosened) using merely one hand by applying a tightening torque to the nut, and a counter-torque to the bolt with the same driver. As another example, this device can be used to apply torque and counter-torque to different components at the same time while mitigating stresses, moments, or torques extending beyond the device, because substantially all stresses, torques and counter-torques are contained within the device. As another example, the driver can be used to screw a fastener into a substrate, while the counter-torque is applied to the substrate, for example screwing a screw into a substrate such as, but not limited to, bone, metal, or wood. As another example, an auger may be used to draw earth from the ground, while the housing of the device is engaged with the ground to apply counter-torque to stabilize the device.

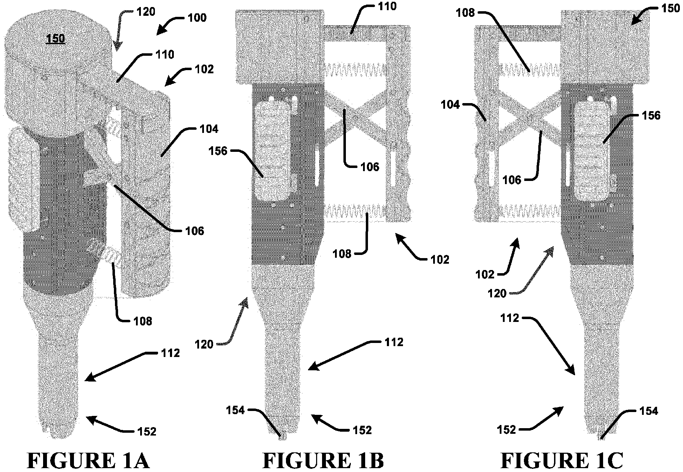

FIGURES 1A-1G are component diagrams illustrating one example implementation of a device 100 for applying torque to a fastener (e.g., a threaded fastener, such as a screw or bolt) in a first direction while applying counter-torque in a second direction. In this way, for example, the threaded fastener can be driven into (e.g., or out of) a target base using the threads of the screw, while rotation of the target base and/or device 100 is mitigated. FIGURE 1A is a isometric view, 1B is a first side view, 1C is a second side view, 1D is a front view, 1E is a rear view, 1F is a top view, and 1G is a bottom view.

As illustrated, the device 100 comprises a manually active actuator 102 disposed at a first end 150 (e.g., proximal end or upper portion) of the housing 120 of the device 100. The actuator 102 comprises a handle 104, actuator guide/support members 106, a biasing spring 108, and an activator gear 110. Further, at a second end 152 (e.g., distal end or lower portion) of the housing 120, a tool driving portion 112 is disposed. As will be disclosed further below, the tool driving portion 112 comprises a rotating component (e.g., shaft) that drives a target tool 154 (e.g., fastener bit), which, for example, may be of any particular design configured to operably interface with the target component (e.g., fastener). Additionally, one or more buttons or switches 156 can be disposed on the sides of the upper portion of the housing 120, and can be used, for example, to select rotation direction of the applied torque, as will be described further below.

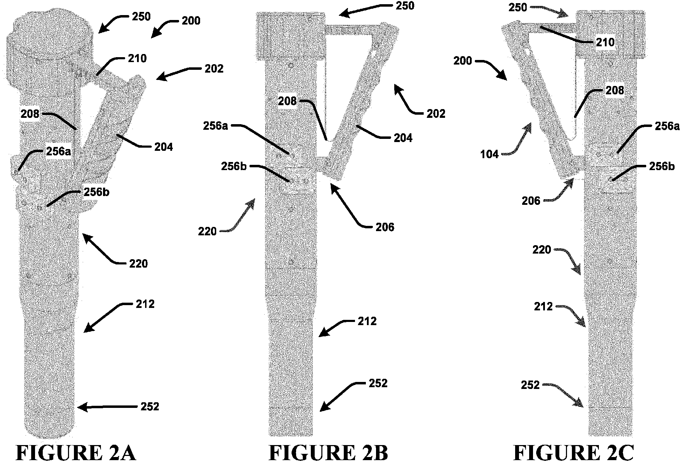

FIGURES 2A-2G are component diagrams illustrating another example implementation of an alternate device 200 for applying torque to a target tool in a first direction while applying counter-torque in a second direction. The device 200 comprises housing 220, which houses various components of the device 200. Further, the device comprises a first end 250 (e.g., proximal end or upper portion) of the housing 220, and a second end 252 (e.g., distal end or lower portion) of the housing 220. Further, in this example implementation of the device 200, an manually operated actuator 202 comprises a handle 204, an actuator hinge/connection point 206, a biasing spring 208, and an activator gear 210. A tool driving portion 212 is disposed at the second end 252. Additionally, one or more buttons or switches 256 (256a, 256b, and so on) can be disposed on the sides of the upper portion 250 of the housing 220 and can be used to select rotation direction of the applied torque, and/or change an amount of torque/rotation applied, as will be described below.

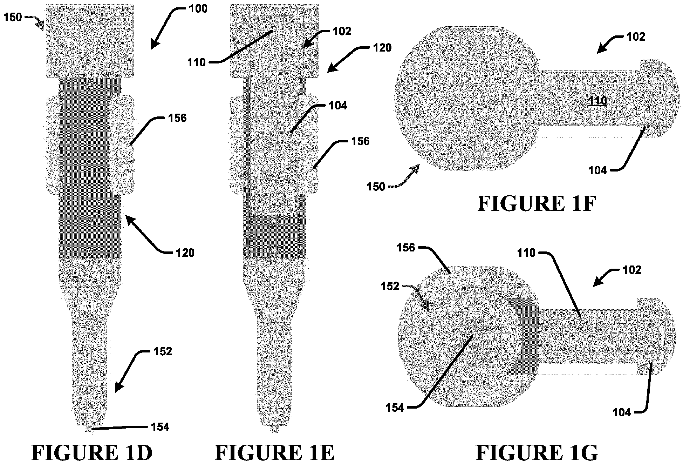

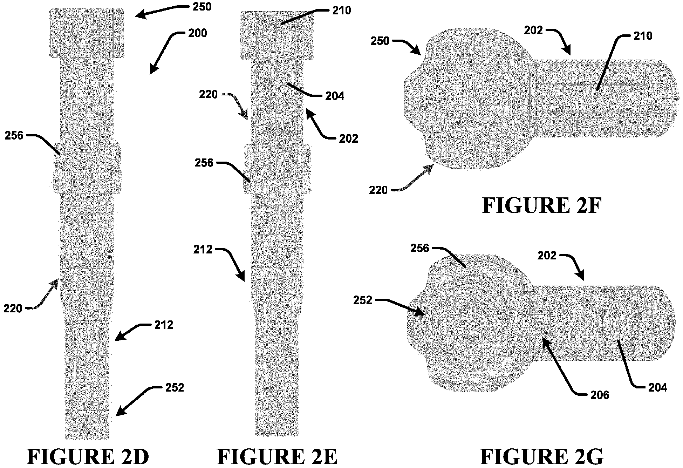

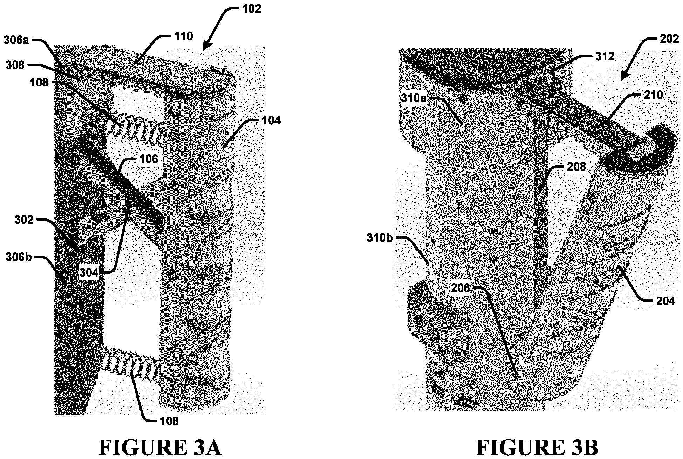

FIGURES 3A and 3B are component diagrams, in a perspective view, of two different alternate designs of the manually operated actuator 102, 202 of the example devices 100, 200. With continued reference to FIGURES 1 and 2, as described above, in FIGURE 3A, the actuator 102 of device 100 comprises a handle 104, guide members 106, biasing springs 108, and the activator gear 110. Further, in this implementation, the guide members 106 are coupled together at a pivot point 304 to provide a scissor-type action. In this implementation, the guide members 106 ride in channels 302 in the lower handle housing 306b of the upper portion 150 of the device 100, for example, and slide up and down when the handle 104 is compressed and released. Additionally, the biasing springs 108 can provide a biasing force away from the lower handle housing 306b to return the handle 104 to its starting position (as shown) when compression is removed. As an example, when the handle 104 is compressed the activator gear 110 is translated into the upper handle housing 306a through an actuator opening 308 in the upper handle housing 306a. In this example, when the handle 104 is released the biasing springs 108 provide the biasing force to return the handle 104, and the activator gear 110 to their starting positions (e.g., as illustrated).

In FIGURE 3B, the actuator 202 of the device 200 comprises a handle 204, a hinge/connection point 206, a biasing spring 208, and an activator gear 210. Further, in this implementation, the hinge/connection point 206 couples the handle 204 with the lower handle housing 3 10b of the device 200 in a pivotal arrangement. In this way, for example, the hinge/connection point 206 can stabilize the handle 204 when it is compressed and returned to its starting position (as shown), while providing a pivot point for the linear force applied to the activator gear 210 by the handle 204. Additionally, as illustrated, when the handle 204 is compressed the activator gear 210 is driven into an opening 312 in the upper handle housing 310a; and, when the compressive force applied to the handle 204 is released the biasing spring 208 applies a biasing force that can return the handle 204 (e.g., and activator gear 210) to its starting (uncompressed) position. As illustrated, in this implementation, the biasing spring 208 can be fixedly engaged with the housing 310a or 310b and with the handle 204. In this example, the biasing spring is formed from a single band suitable material (e.g., metal, polymer, carbon fiber, etc.) disposed in a "U" or "V" shape. However, it is anticipated that any suitable design may be employed, where the biasing force is applied in a direction that operably separates (e.g., drives apart) the handle 204 from the housing 310a.

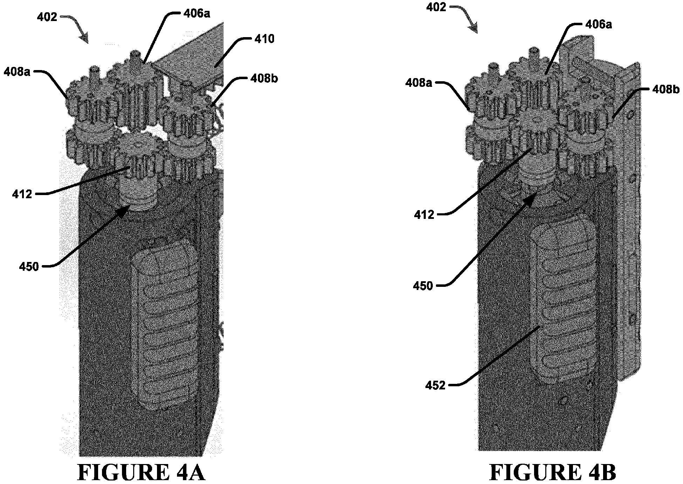

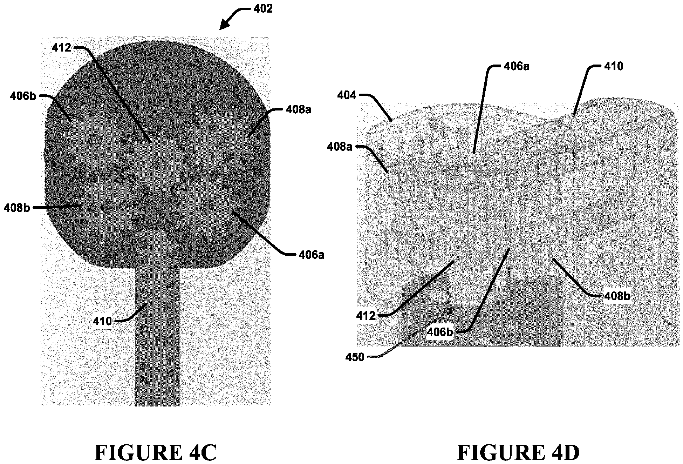

FIGURES 4A-4D are component diagrams that illustrate various example implementations of a power input drive 402 in a first position (e.g., with a center rack gear 410 shown in FIGURES 4A, 4C, 4D), and a second position (shown in FIGURES 4B, center rack 410 not shown for better visibility). In this implementation, power input drive 402 is of a rack and spur gear design, with a slidable center rack gear 410 (e.g., the activator gear 110, 210), which is attached to and translates linearly within the ratchet mechanism box 404 portion of the upper handle housing (e.g., 306a, 3 10a); and further engages with one or more spur gears (such as 406a, 406b (not shown for better visibility), 408a, 408b). Further, the rack and spur gear design of the power input drive 402 can be combined with reversible mechanism that can reverse the direction of rotation of the drive, as shown in Figure 4B, by translating switch 452 (e.g., or 156) upward. In this way, the driving of the center rack gear 410 into the power input drive 402, for example, can result in the drive rotating clockwise in a first position of switch 452 and thereby drive shaft gear 412 (FIGURES 4A, 4C, 4D), and counterclockwise in a second position (FIGURE 4B).

In these examples, when the center rack gear 410 is driven into the power input drive 402 (e.g., from the first position to the second position), the gear teeth on the center rack gear 410 meshably engage with one or more initial spur gears 406a, 406b. In this example, as illustrated in FIGURES 4A, 4C and 4D, in a first position, the initial spur gears 406a, 406b will rotate, respectively, in a clockwise and counterclockwise direction. Spur gear 406a meshably engages secondary spur gear 408a resulting in a counterclockwise rotation. Finally, the lower portions of initial spur gear 406b and secondary spur gear 408a meshably engage with drive shaft gear 412 resulting in the drive shaft 412 to be rotated in a clockwise direction, when the handle 104 is compressed. As illustrated in FIGURE 4B, the drive shaft gear 412 can be disposed in a second position, such as an up position, which may be selected by activating (e.g., translating up) a selection switch 452 (e.g., 156 of FIGURE 1). In the second position, the drive shaft gear 412 is disengaged from the secondary spur gear 408a, and initial spur gear 406b, and is meshably engaged with the initial spur gears 406a, and secondary spur gear 408b, which is meshably engaged with initial spur gear 406b. In this way, when the gear teeth on the center rack gear 410 rotate the initial spur gear 406a and secondary spur gear 408b, respectively, in the clockwise and counterclockwise direction, the meshably engaged drive shaft gear 412 will be rotated in the counterclockwise direction, when the handle 104 is compressed.

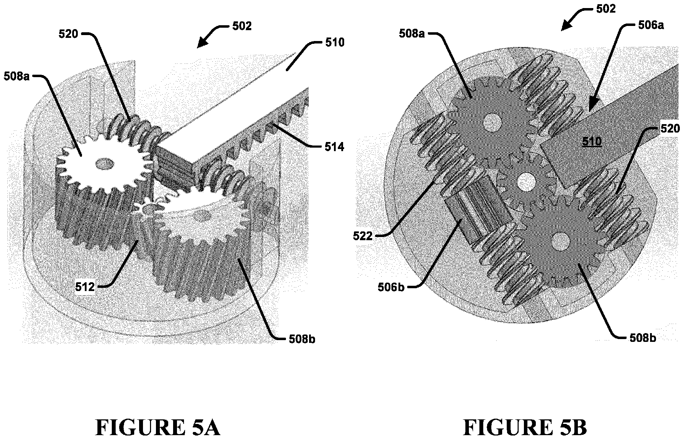

FIGURES 5A and 5B show an alternate arrangement of a power input drive 502. As illustrated, in this implementation, the gear teeth 514 of the center rack gear 510 meshably engage with a first initial spur gear 506a that rotates in a horizontal axis. The first initial spur gear 506a is centrally fixed to a first worm gear 520 that rotates around the horizontal axis of the first initial spur gear 506a. As such, when the center rack gear 510 is driven into the power input drive 502 (e.g., by compressing the handle 104) the first initial spur gear 506a and first worm gear 520 rotate in a first direction (e.g., counterclockwise when viewed from a side view of the thread of the worm gear). The first worm gear 520 is meshedly engaged with one or more secondary spur gears 508a, 508b, which will rotate in a counterclockwise direction when the first worm gear 520 rotates in the first direction. A drive shaft gear 512 is disposed between the secondary spur gears 508a, 508b, and rotates in a clockwise direction, which in turn rotates the drive shaft in a clockwise direction, when the handle 104 is compressed.

Alternately, as illustrated in FIGURE 5B, a second worm gear 522 with a second initial spur gear 506b can be meshedly engaged with the center rack gear 510, such by activating a selection switch or button, which simultaneously deactivates the first worm gear 520. In this example, when the second worm gear 522 is activated, the second initial spur gear 506b rotates in a clockwise direction, which rotates the second worm gear 522 in a clockwise direction, which, based on the thread arrangement, results in the secondary spur gears 508a, 508b rotating in a clockwise direction. In this way, the engaged drive shaft gear 512 will rotate in a counterclockwise direction, as will the drive shaft, when the handle 104 is compressed. It may be appreciated that a power source may be powered manually, by electric motor, pneumatic drive, hydraulic drive, or any other suitable means.

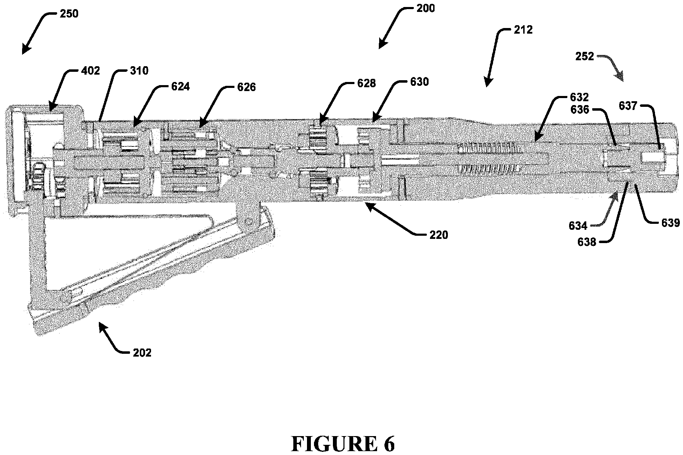

FIGURE 6 is a cutaway view of the example tool driver device 200. The example device 200 comprises the first end 250, the second end 252, the actuator 202, power input drive 402 (e.g., or 502), and housing 220, as described above. Further, the example device 200 comprises a ratcheting mechanism 624, a rotation reversal mechanism 626, a torque conversion mechanism 628, a torque limiter 630, a rotating drive shaft 632 (e.g., retractable), and a counter-torque component holder 634 (e.g., sleeve for holding a counter-torque tip), along with a drive tip holder 636 (e.g., which may be formed as a part of the rotating shaft 632, or a separate component at the distal end of the shaft). The counter torque component holder 634 can comprise a distal end 638 that is formed to operably hold a counter torque component 639 (e.g., counter-torque tool, such as a female hex-shaped). The drive tip holder 636 and counter torque component holder's distal end 638 can be configured to selectably accept different types of driver tools 637 and counter torque component 639 (e.g., tools), respectively. As an example, the rotating drive shaft 632, using the drive tip holder 636, can selectably engage with a first drive tool, and selectably engage with a second, different drive tool. Further, the counter torque component holder 632 can selectably engage with a first counter-torque component (e.g., tool), and selectably engage with a second, different counter-torque component (e.g., tool).

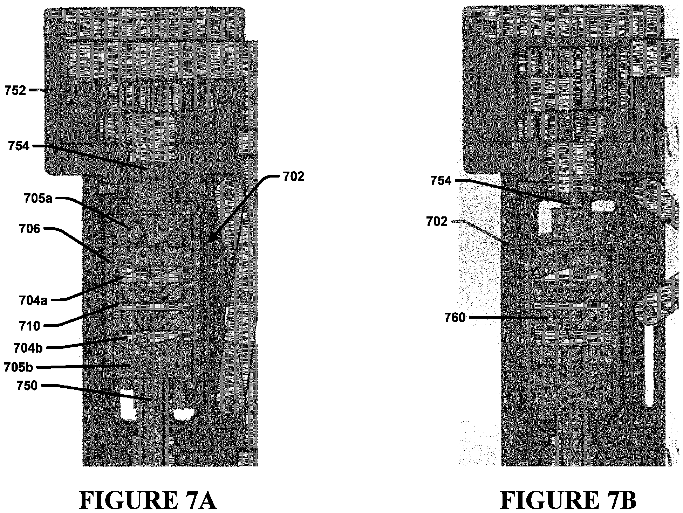

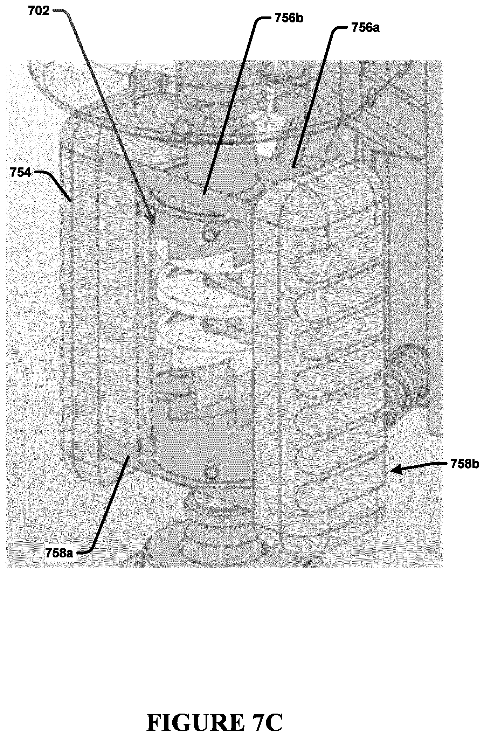

In some implementations, a ratcheting mechanism 624 may be provided to allow rotation in a first direction while mitigating rotation in a second direction. In one implementation, as illustrated in FIGURES 7A-7C, a ratcheting mechanism 702 may be selectably reversible, to allow for rotation in either rotational direction (e.g., while mitigating opposite rotation). FIGURE 7A shows the ratcheting mechanism 702 in a first position, and FIGURE 7B and 7C show the ratcheting mechanism in a second position. In the first position, in this example, the drive shaft 754 from the power input drive 752 (e.g., 402, 502 in FIGURES 4 and 5) can be rigidly connected to the casing or body 706 of the ratcheting mechanism 702, and thereby drive pawls 705a and 705b. In the first position the driving pawl 705b of the ratcheting mechanism 702 rotates the lower driven pawl 704b, which then drives the drive shaft 750 in a counterclockwise direction. When the ratcheting mechanism 702 is selectably switched to the alternate position, for example, by translating the switch 156 to an upward position (e.g., first position in FIGURE 7A), the drive shaft 750 is allowed to rotate in a counterclockwise rotation (e.g., unscrewing). When the ratcheting mechanism 702 is selectably switched by translating the switch 156 to a downward position (e.g., second position in FIGURE 7B and 7C), the upper driving pawl 705a of the ratcheting mechanism 702 rotates the upper driven pawl 704a, and the drive shaft 750 is allowed to rotate in a clockwise rotation (e.g., screwing in or driving into a target component). Wave springs or washers 760 can be disposed between a static disc 710 and the upper/lower "driven" pawls 704a, 704b, to allow ratcheting in the desired direction of rotation due to teeth on discs.

As illustrated in FIGURE 7C, a slide switch 754 (e.g., 156 of FIGURE 1, or the like) can be activated by sliding up or down. In this implementation, the slide switch 754 is fixed to a pair of pins above 756a, 756b and below 758a, 758b the ratcheting mechanism 702. The pins 756a, 756b, 758a, 758b are engaged with ratcheting mechanism 702 to translate it within the housing, e.g., up and down) along the drive shaft which may also move the drive shaft gear (e.g., 412), for example, up and down in the power input drive 402 or 752.

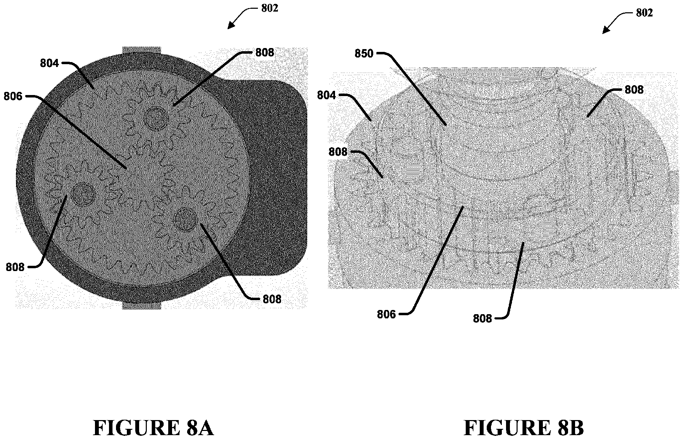

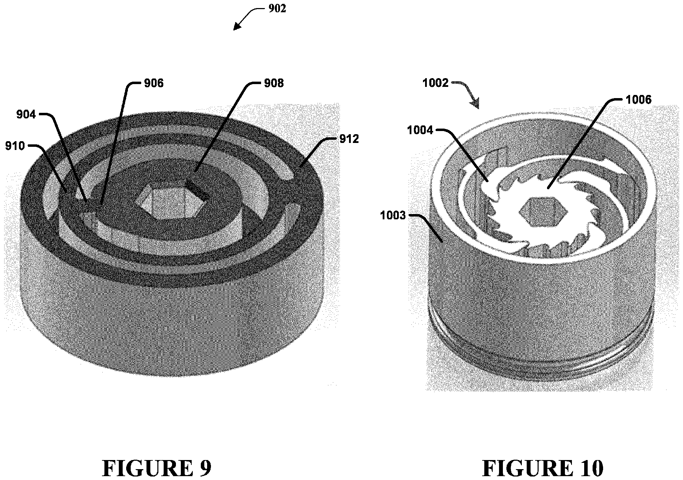

As illustrated in FIGURE 10, an alternate ratcheting mechanism 1002 may be utilized. In this implementation, the inner spur gear 1006 is driven by the power drive shaft (e.g., 754 of FIGURE 7A, 7B; or 750) from the power input drive 402 (e.g., or 502 or 752). Multiple flexible tabs 1004 can engage and transfer the rotational torque to the outer housing 1003, which is rigidly connected to the drive shaft (not shown, e.g., 850 of FIGURE 8B). In some implementations, the ratcheting mechanism 1002 can be inverted, so that the outer housing 1003 is driven by the power drive shaft and the inner spur gear 1006 is connected to the drive shaft.

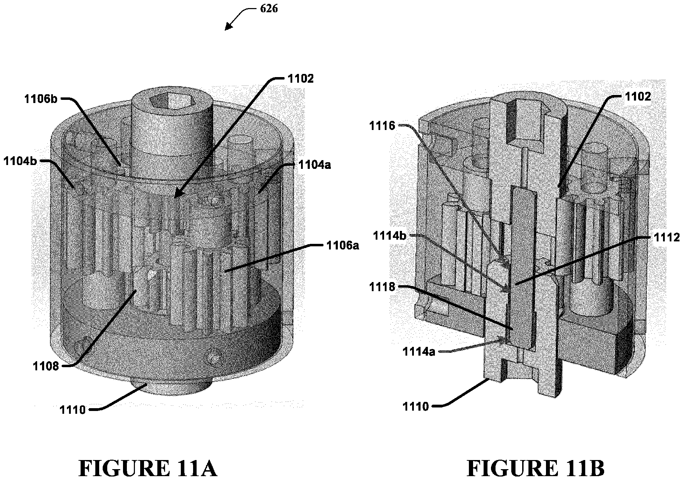

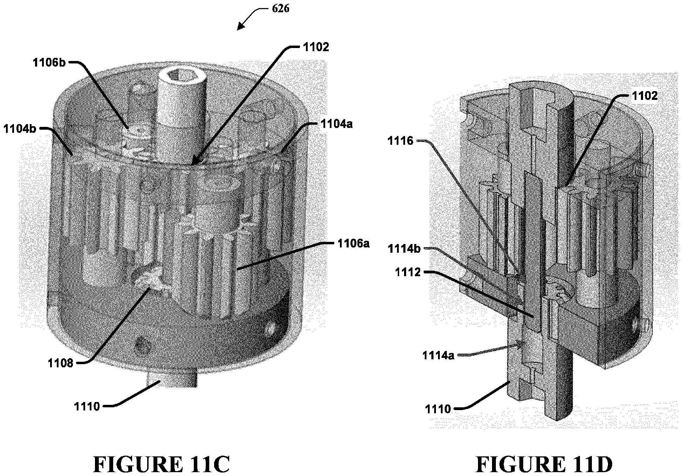

In some implementations, the example device 200 can comprise a rotation reversal mechanism 626 as shown in FIGURE 11A, 11B, 11C, 11D. In this implementation, the rotation reversal mechanism 626 can comprises a input center gear 1102, which is driven by an input torque, two or more upper spur gears 1104a and 1104b, two or more lower spur gears 1106a and 1106b and a slidable center gear 1108 that can translate between an up and down position. The input center gear 1102 is meshedly engaged with the upper spur gears 1104a, 1104b, which are meshably engaged with the lower spur gears 1106a, 1106b. In some implementations the input center gear 1102 can comprise an extension 1112 with a medial cylindrical portion 1118a and a shaped end portion 1118b (e.g., a hex-drive extension), which is disposed inside a recess 1116 of the second drive shaft 1110. The recess has an annular inner recess 1114a disposed medially; and a shaped female feature 1114b at its end, which is complementary to the shaped end 1118b. As illustrated in FIGURE 11A and 11B, in an up position of the second drive shaft 1110 the second drive gear 1108 is meshably engaged with the lower spur gears 1106a and 1106b resulting in a reverse rotational direction of 1110 compared to 1102. In this example, the shaped portion 1118b of the extension 1112 of input center gear 1102 is disposed inside the medial annular inner recess 1114b of the second drive shaft 1110, which allows it to rotate freely without engaging with the second drive shaft 1110. Further, as illustrated in FIGURE 11C and 11D, with the second drive shaft 1110 (e.g., and center gear 1108) in a down position, the second gear drive 1108 is not meshably engaged with the lower spur gears 1106a and 1106b, but the shaped portion 1118b of the extension 1112 of the input center gear 1102 is disposed inside the complementary-shaped female feature 1114a of the second drive shaft 1110 resulting in engagement and transfer of force between driveshaft 1102 and driveshaft 1110, in a same rotational direction.

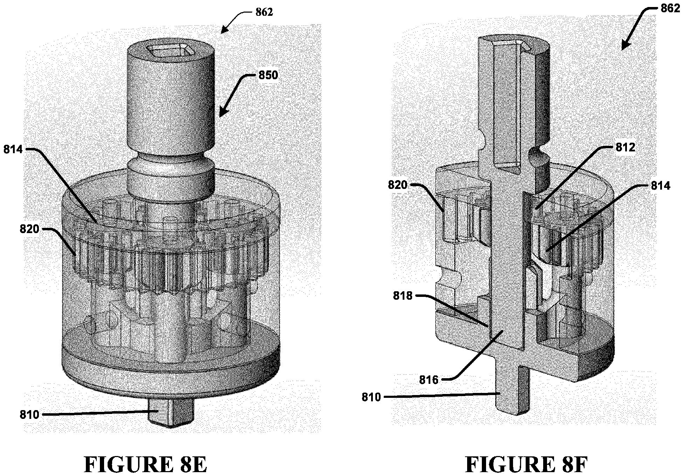

In some implementations, an example device can comprise a torque conversion mechanism (e.g., 628 of FIGURE 6). FIGURES 8A-8H are component diagrams illustrating some example implementations of torque conversion mechanisms 802, 860, 862, which allows for selecting a torque output ratio with regard to torque input. As an example, the torque conversion mechanism 802 can comprise a planetary gear arrangement. For example, the purpose of this gear arrangement is to provide an input to output torque conversion, such as 1:1, 1:3, 1:4, etc. That is, in example 802, a ring gear 804 can be static, and a sun gear 806 can be driven with torque applied by the drive shaft 850 (e.g., connected to the driven pawl of the ratcheting mechanism 704b, 1006, or the secondary drive shaft of the rotation reversal mechanism 626). In this example, this results in planetary gears 808 orbiting the sun gear 806, providing rotation to a driver shaft 810. When engaged, for example, the torque output can comprise a 4-fold increase in torque, as illustrated in implementation 862 of FIGURES 8E-H with 4 planetary gears, or a 3-fold increase as illustrated in implementations 802, 860 of FIGURES 8A-D with three planetary gears (e.g., or some other ration depending on the number of teeth, size, and arrangement of gears).

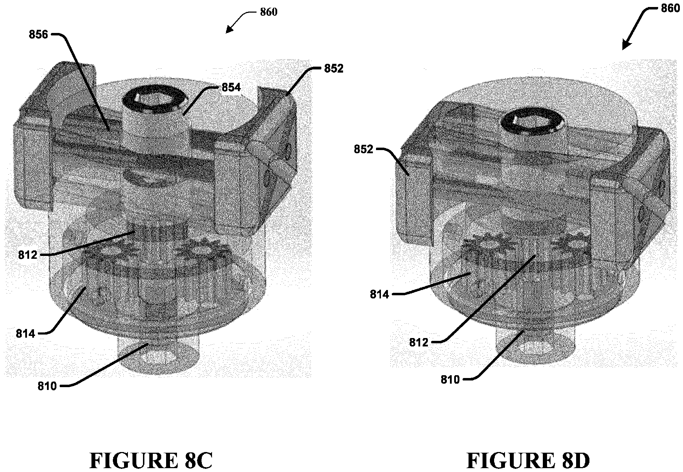

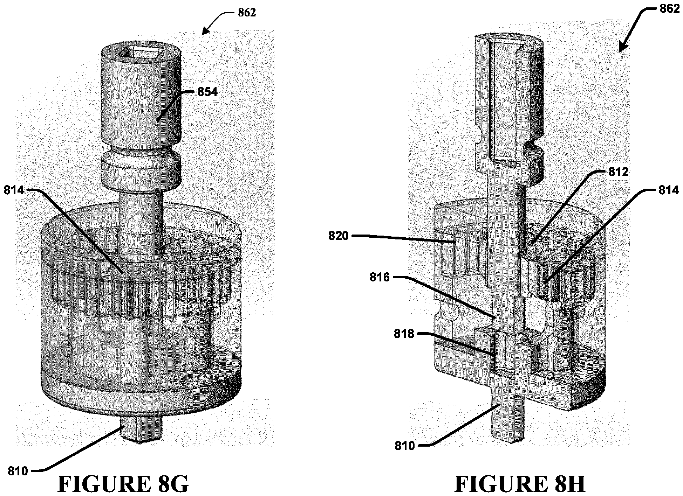

In an alternate arrangement, as illustrated in FIGURES 8C-H, a bypass/switching option can be used, for example, to speed up initial tightening of the target fastener. That is, for example, the torque convertor 860, 862 can be switched between the 1:4 (e.g., or 1:3) torque input to output (e.g., which results in a slower rotation and 4-fold higher output torque of the driven shaft 810), to a 1:1 torque input to output, resulting in a faster rotation with the same output torque of the driven shaft 810. In this example, the example torque converter 860, 862 comprise a sun gear 812, planetary gears 814, a ring gear 804 or 820 (not shown in FIGURE 8C, 8D), and a sliding shaft 812. In this implementation, a switch 852 can be engaged with a slidable sleeve 854 by attached pins 856. The sleeve 854 can be engaged with the central sun gear 812 to move it into and out of engagement with the planetary gears 814. When the sun gear 812 is disposed in engagement with the planetary gears 814, as in FIGURE 8G, 8H, the 1:4 torque ratio is employed. In this example, when the sun gear 812 is disposed out of engagement with the planetary gears 814, as in FIGURE 8E, 8F, the 1: 1 torque ratio is employed, because the male drive 816 directly engages the female feature 818. Further, the torque ratio may be adjust using different arrangements, numbers, and dimensions of torque converter components.

In one implementation, an example device (e.g., 200) can comprise a torque limiter (e.g., 630 of FIGURE 6). For example, as illustrated in FIGURE 9, a torque limiter 902 can be configured to provide a predetermined output of torque, which can be limited to desired torque value. As an example, the torque limiter 902 can comprise a "ring-spring" torque limiter, which is operable for a predetermined amount of torque, and precision machined ring-spring 910 with a tab 904, which is operably disposed in a groove 906 of a central drive shaft engagement sleeve 908. In one example, the ring-spring 910 can be machined in such a way as part of the housing 912 where a predetermined amount of torque is utilized to rotate the tab 904 out of the groove 906. As an example, when the amount of torque applied to the drive shaft, and hence the shaft sleeve 908, the torque will be transferred from groove 906 to tab 904 and thereby to the output shaft (not shown), which is rigidly connected to the housing 912. If the torque transfer exceeds the predetermined allowable torque transfer value between tab 904 and groove 906, then the tab 904 will translate out of groove 906 (e.g., producing an audible "click"), which indicates the preselected amount of torque has been reached. In this example, because the tab 904 is no longer disposed inside the groove 906, no additional torque will be transferred. It is anticipated that other suitable torque limiting mechanisms are also contemplated for use in the device. For example, the use of an electro-mechanical torque limiter is anticipated for devices using an electric motor as a power source. In those implementations the current draw by the electrical motor can be monitored in order to turn off the motor, when the current draw has reached a value that correspond to a predetermined torque level.

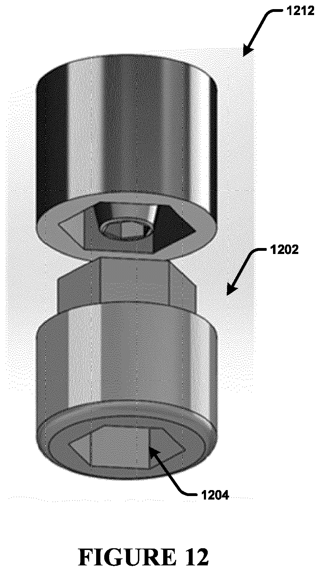

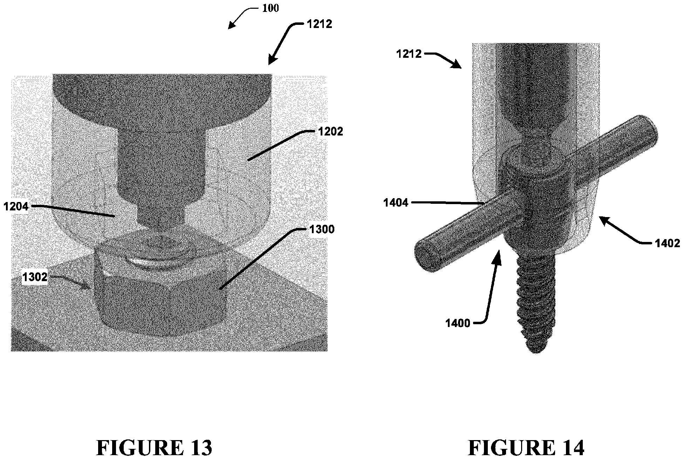

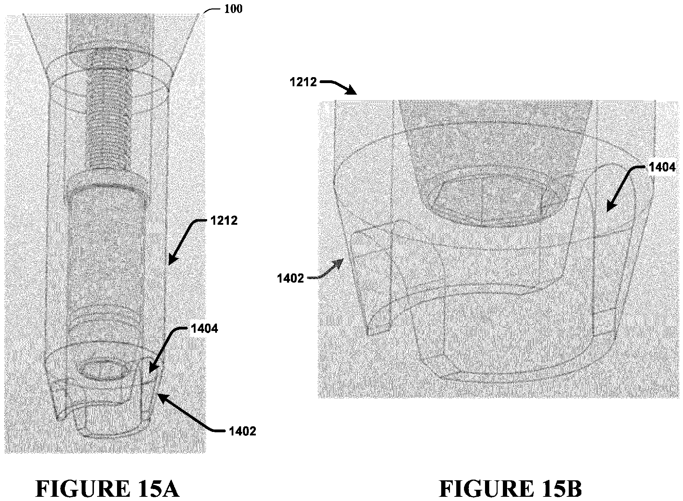

As illustrated in FIGURE 12, a counter-torque component 1202 (e.g., tool tip) can be selectably engaged (e.g., selectably fixed and removed/replaced) with the counter-torque component holder portion 1212 (e.g., 212) of an example device. As an example, as illustrated in FIGURES 13, 14, 15A, and 15B, a target base 1300, 1400 (e.g., into which a target fastener is driven and/or removed) can comprise a variety of shapes and sizes. In this implementation, a counter-torque tool 1202, 1402 (e.g., the component formed to engage the target base) can be configured to engage/fit the target base 1300, 1400. As illustrated, for example, counter-torque tool 1202 has a hex-shaped cavity 1204 that is configured to operably fit over the hex-shaped protrusion (e.g., nut) of the target base 1300. Further, for example, the counter-torque tool 1402 has a slot-shaped cavity 1404 that is configured to operably fit over the bar portion of the target base 1400 (e.g., a spinal stabilization bar). In this way, for example, the appropriate counter-torque tool can be fixedly engaged with the counter-torque component holder portion of the device and hold the device stationary with respect to the target base, while a fastener is driven into, and fixed to, the target base. It is anticipated that various shapes, sizes and configuration of counter-torque sleeves can be design depending on the configuration of the target component.

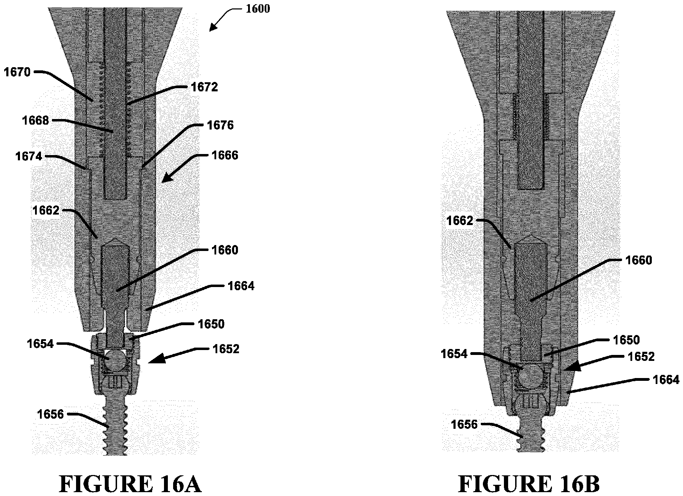

FIGURES 16A and 16B are component diagrams illustrating an example implementation of a portion of an example device 1600, where a target fastener 1650 (e.g., a set screw) is operably engaged with a target base 1652 (e.g., a spinal stabilization apparatus). In this example, the target fastener 1650 can comprise a set screw that can be operably tightened or loosened into the target base 1652, which is used to secure a spinal stabilization rod 1654 to a pedicle screw 1656. As illustrated, a selectably removable fastener bit tool 1660 can be chosen to operably accommodate the head of the fastener 1650. The bit tool 1660 is engaged into the bit holder 1662 of the device. An appropriate counter-torque tool 1664 is selected and fixedly engaged onto the end of the counter-torque component holder portion 1666. In this example, the counter-torque tool 1664 can be selected with a slot-shaped groove or cavity to operably fit over the stabilization rod 1654. As illustrated, the fastener bit tool 1660 is operably engaged with the fastener 1650 in FIGURE 16A. In FIGURE 16B, the counter-torque tool 1664 can be operably fit over the target base 1652, in this case the stabilization rod 1654 portion. In other implementations, the counter-torque tool 1664 can be configured to operably fit over a different target base, such as the housing of a pedicle screw system.

In this implementation, the example device 1600 comprises a bit holder 1662 and driven rotating shaft 1668, that can translates up and down inside a cavity 1670 in the counter-torque component holder portion 1666. Further, a biasing spring 1672 is disposed inside the cavity 1670 to provide a biasing force against the bit holder 1662, toward the distal end of the device 1600. In this way, the biasing force drives the bit holder 1662 toward the distal end (e.g., second end). A bit holder stop 1674 is disposed at the distal end of the cavity 1670 to engage with a shoulder 1676 on the bit holder 1662. In this way, for example, the biasing force will provide for the removable fastener bit initially protruding from the counter-torque sleeve 1664 for easier insertion of the fastener bit tool 1660 into the fastener, while holder stop 1674 stops the bit holder 1662 from being pushed out of the counter-torque tool 1664.

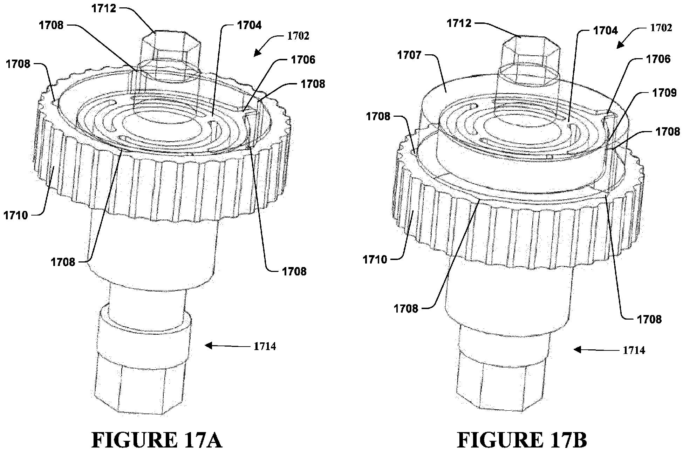

FIGURES 17A and 17B illustrate another example implementation of a torque limiter (e.g., FIGURE 9). As illustrated in FIGURE 17A, a torque limiter 1702 can be configured to provide a predetermined limit of torque output (e.g., optionally pre-selected), which can be preselected at different desired torque values. As an example, the torque limiter 1702 can comprise a drive shaft 1712, a housing 1707 for a predetermined (e.g., amount of torque), and precision machined ring-spring 1704. An integrated pin 1706 can be operably disposed inside a groove 1709 of housing 1707 and protrude into a recess 1708 of an outer ring 1710. In this implementation, one or more recesses 1708 (in this example five) can be disposed in the outer ring 1710, each having a different sized/shaped cavity (e.g., with different depths, wall angles, etc.), thereby creating different tangential forces to discharge the pin 1706 at a predetermined torque.

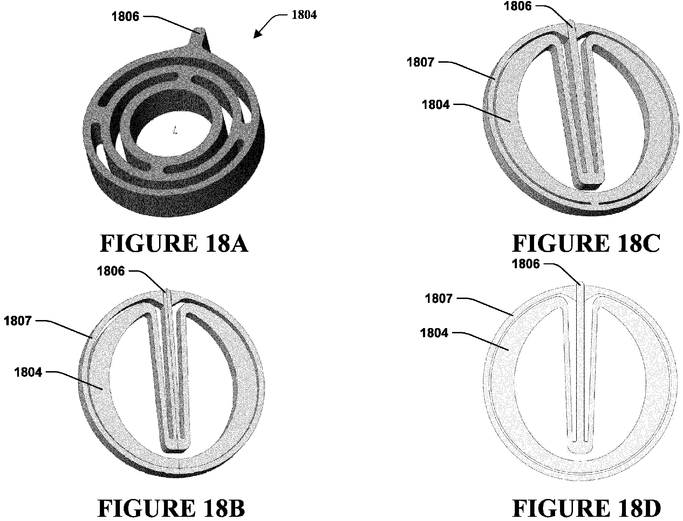

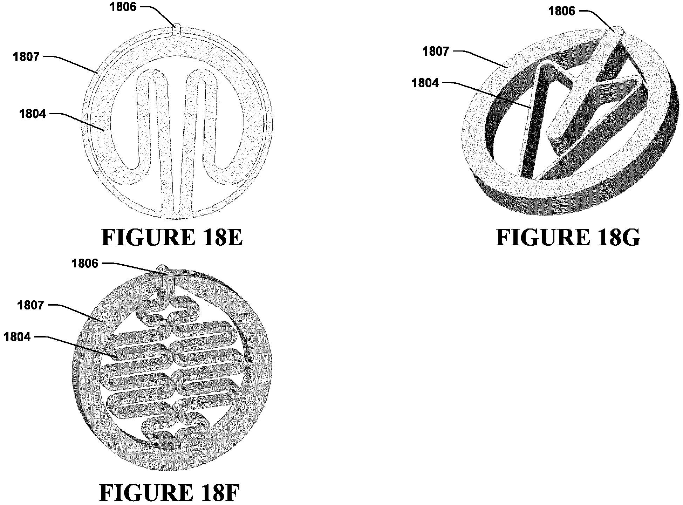

As an example, the biasing force of the ring-spring 1704 is substantially constant (e.g., predetermined by design, illustrated below in FIGURES 18), and the recesses 1708 each have different shapes/sizes designed for different torque values. In this example, the pin 1706 is operably disposed into a pre-selected recess. Thus, the torque value that results in the pin 1706 discharging out of the selected recess 1708 is determined by the different depths and wall angles of a recess 1708, and the associated tangential force. As an example, when torque is applied to the drive shaft 1712, and hence to the housing 1707, the torque will be transferred from the housing 1707 to the pin 1706 and then from the pin 1706 to the selected recess 1708, and thereby to the outer ring 1710. The outer ring 1710 is rigidly connected in rotation to the output shaft 1714. Therefore, in this example, if the torque transfer is below the predetermined threshold torque transfer value between the pin 1706 to the selected recess 1708, then the outer ring 1710 and output shaft 1714 will rotate with the same input torque as drive shaft 1712. Further, when the torque transfer exceeds the predetermined threshold torque transfer value between the pin 1706 to the selected recess 1708, the pin 1706 will dislodge from the selected recess 1708 and subsequent rotation is mitigated, at least until reset. In this example, the dislodging of the pin 1706 can produce an audible "click," which indicates to the user that the preselected amount of torque has been reached. Further, because the pin 1706 is no longer seated inside the selected recess 1708, torque is no longer be transferred. It is anticipated that other suitable torque limiting mechanisms are also contemplated for use in the device.

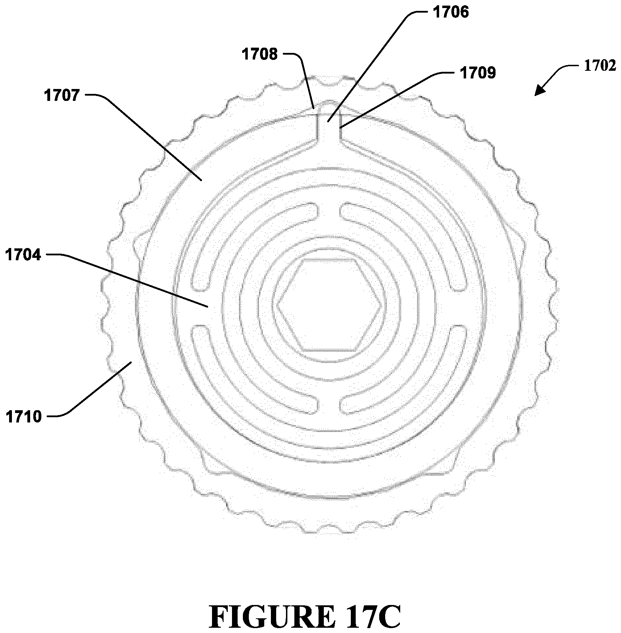

As illustrated in FIGURE 17B, as an example, when selecting the appropriate recess 1708 in the outer ring 1710 for the position of the pin 1706 (e.g., and thus pre-selected torque value), the outer ring 1710 may be axially pushed down, which disengages the pin 1706 from the outer ring 1710, and the outer ring 1710 from the output shaft 1714. In this example, the outer ring 1710 can be rotated to another selected recess 1708 and released, such that the outer ring 1710 is axially spring loaded upward. In this example, the resulting new engagement between pin 1706 and the newly selected recess 1708 can create a rotational engagement between outer ring 1710 and output shaft 1714. As an example, in FIGURE 17C, a top view of torque limiter 1702 illustrates a ring-spring 1704 with an integrated pin 1706, which is inserted through a groove 1709 of housing 1707 and further protrudes into a recess 1708 of an outer ring 1710.

In other implementations of a ring-spring (e.g., 1704 in FIGURES 17A, 17B, 17C), for example, as illustrated in FIGURE 18A, a ring-spring has a predetermined (e.g., threshold amount of torque) and precision machined ring-spring 1804 with pin 1806 either integrated or seated inside a housing 1807. Also as illustrated in FIGURES 18B, 18C, 18D, 18E, 18F, and 18G, there are multiple varied configurations of the ring-spring 1804, each machined with a predetermined amount of torque and a pin 1806 configured to engage a recess (not shown). Those skilled in the art would appreciate these and other suitable ring-spring configurations. In other implementations, for an electro-mechanical torque limiter, an operator can define a desired torque limit, for example, with a dial, digital input, touchscreen input, etc., which thereby defines a predetermined set-point for the current draw that can trigger the motor to shut off.

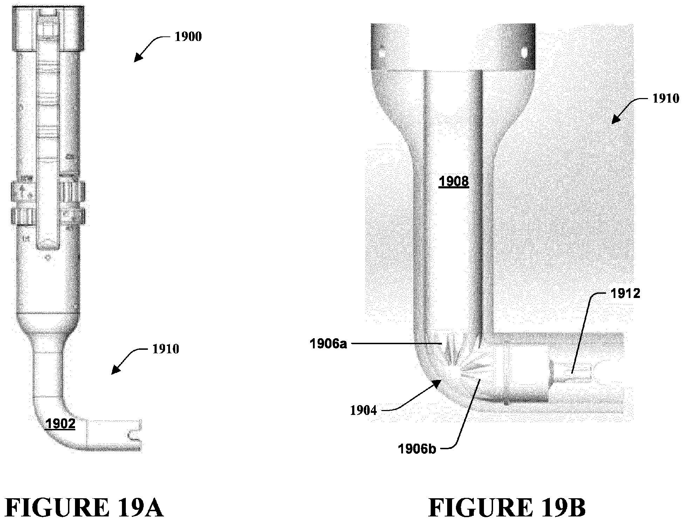

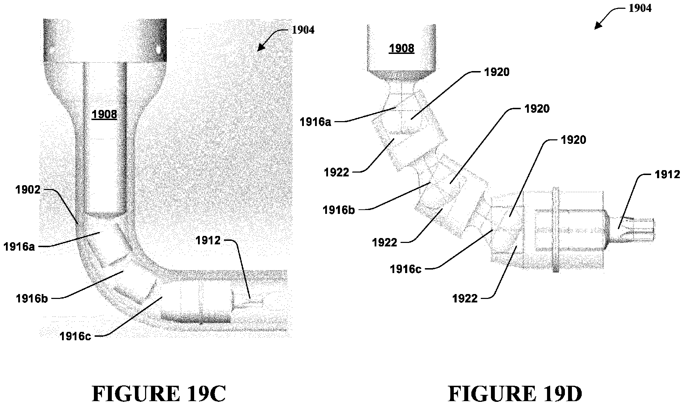

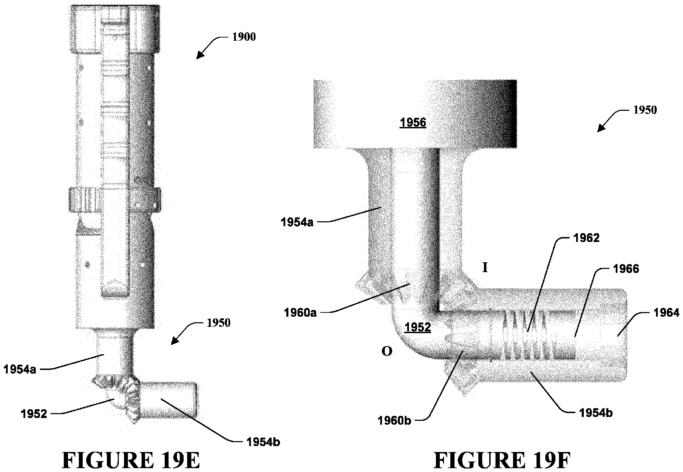

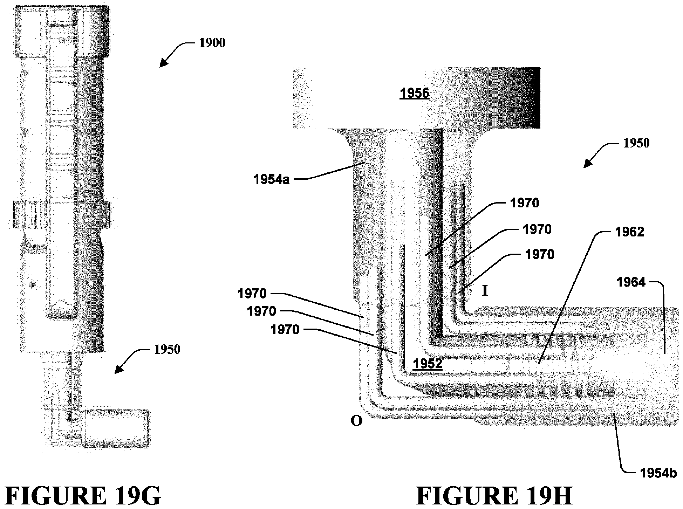

In FIGURES 19A, 19B, 19C, 19D, 19E, 19F, 19G, and 19H are component diagrams illustrating an example implementation of a portion of an example device 1900, namely an angled shaft 1910, comprised of a cover 1902 and rotating inner shaft components 1904. The example device 1900 may be powered manually, by electric motor, pneumatic drive, hydraulic drive, or any other suitable means. The diagram angles are shown as 90 degrees, but it is contemplated that it may be configured to any suitable angle. As illustrated in FIGURE 19A, an implementation of the angled shaft 1910 may include a cover 1902 that contain the rotating inner shaft components 1904. As shown in FIGURE 19B the rotating inner shaft components 1904 comprise a pair of bevel gears 1906a, 1906b. As illustrated in this implementation, the bevel gears 1906a and 1906b are meshedly engaged at a 90-degree angle, however it is appreciated that the bevel gears 1906a and 1906b may be machined to engage at any suitable angle. As output shaft 1908 rotates clockwise or counterclockwise so does bevel gear 1906a, as it is fixedly attached to output shaft 1908. As bevel gear 1906a rotates, bevel gear 1906b rotates in the same direction thus rotating the fastener driving portion 1912 also in the same direction.

FIGURES 19C and 19D illustrate alternate implementation of a device that applies torque and counter-torque. In these implementations, an angled driver 1910 may include a cover 1902 (not shown in 19D), where the rotating inner shaft components 1904 are at least two (three are shown) round-ball-hex joints 1916a, 1916b, and 1916c. Each round-ball-hex joint 1916a, 1916b, and 1916c comprises a spherical-like hex-ball 1920 and a hexagonal socket 1922, where each hex-ball 1920 is disposed into its associated hexagonal socket 1922. Each hex-ball 1920 is made up of small flat planes that create the spherical-like shape, when disposed into the hexagonal socket 1922 some of the small flat planes fully contact the inner surface of the hexagonal socket 1922 such that torque applied the hex-ball 1920 directly transfers to the hexagonal socket 1922. The hex-ball 1920 of round-ball-hex joint 1916a is fixedly attached to the output shaft 1908, such that torque applied to the output shaft 1908 transfers to the hex-ball 1920 of round-ball-hex joint 1916a.

Thus, in this example, torque applied to the hex-ball 1920 of round-ball-hex joint 1916a, transfers to the hexagonal socket 1922 of round-ball-hex joint 1916a. The hexagonal socket 1922 of round-ball-hex joint 1916a is fixedly attached to the ball 1920 of round-ball-hex joint 1916b. Thus, torque applied to the ball 1920 of round-ball-hex joint 1916b, transfers to the hexagonal socket 1922 of round-ball-hex joint 1916b. The hexagonal socket 1922 of round-ball-hex joint 1916b is fixedly attached to the ball 1920 of round-ball-hex joint 1916c. Thus, torque applied to the hex-ball 1920 of round-ball-hex joint 1916c, transfers to the hexagonal socket 1922 of round-ball-hex joint 1916c. The hexagonal socket 1922 of round-ball-hex joint 1916c is fixedly attached to the bit 1912. The angles in the diagrams are shown as 90 degrees, but it is contemplated that it may be configured to any suitable angle. It may be appreciated that cover 1902 (not shown) is made of an appropriate flexible material containing the round-ball-hex joints 1916a, 1916b, and 1916c that are flexible thereby the angled driver 1910 has an impermanent flexible angle.

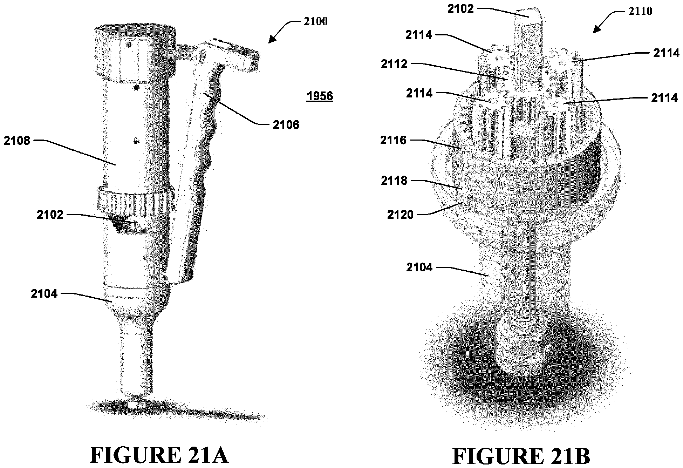

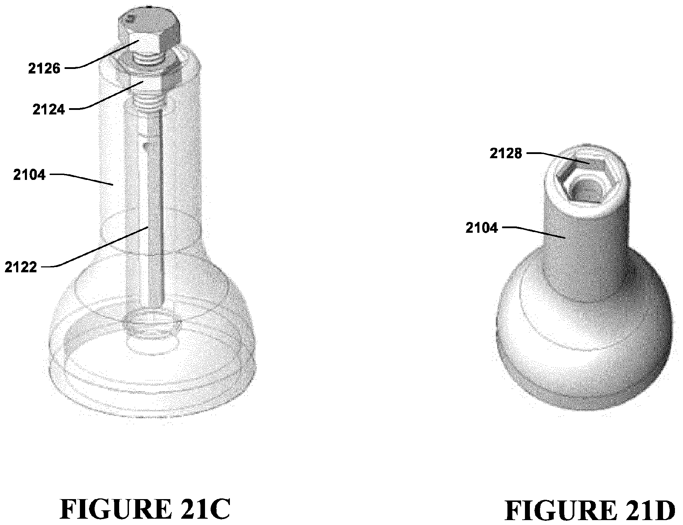

In another implementation, an example device 1900 is illustrated in FIGURES 19E, 19F, 19G, and 19H. An angled driver 1950 comprises an operably, stationary inner shaft 1952 and a rotating outer shaft 1954a and 1954b (e.g., tube). In this example, the arrangement allows for an example nut and bolt-type fastening arrangement (described in detail below with FIGURES 21). As an example, as shown in FIGURES 19E and 19F, the angle driver 1950 comprises an inner (non-rotating) shaft 1952, an outer rotating shaft 1954a and 1954b (e.g., tube) with a pair of bevel gears 1960a and 1960b respectively. In this implementation outer rotating shaft 1954a is fixedly attached to output shaft 1956 and contains a first portion of stationary inner shaft 1952. Outer rotating shaft 1954b has a nut receiver 1964 and contains a second portion of stationary inner shaft 1952 that is a spring 1962. The distal end of the spring 1962 has a bolt receiver 1966.

The stationary inner shaft 1952 is disposed through the outer rotating shaft 1954a and 1954b such that the stationary inner shaft 1952 is disposed at an angle (shown as 90 degrees in this implementation). The stationary inner shaft 1952 bend has an inner corner I and an outer corner O, where the pair of bevel gears 1960a and 1960b meshedly engage at inner corner I. It is appreciated that the stationary inner shaft 1952 and bevel gears 1960a and 1960b may be machined to be disposed through and engage at any suitable angle. In this implementation, as the output shaft 1956 rotates, so does the outer rotating tube 1954a and bevel gear 1960a. As bevel gear 1960a rotates it meshedly engages bevel gear 1960b to rotate in the same direction thus also rotating the outer rotating shaft 1954b and the nut receiver 1964 in that same direction. As the example device 1900 is powered by suitable means, the outer rotating tube 1954b rotates as described above and thereby the nut receiver 1964 applies torque to a target fastener (not shown), while the distal end 1966 of the spring 1962 applies a counter torque to a target component, such as 2126 in FIGURE 21C.

An example is shown in FIGURES 19G and 19H, the angle driver 1950 comprises an inner (non-rotating) shaft 1952, an outer rotating shaft 1954a and 1954b (e.g., tube) with a plurality of slidable rods 1970 where respective slidable rods are substantially uniform in size. In this implementation outer rotating shaft 1954a is fixedly attached to output shaft 1956 and contains a first portion of stationary inner shaft 1952. Outer rotating shaft 1954b has a nut receiver 1964, contains a second portion of stationary inner shaft 1952 that is a spring 1962. The distal end of the spring 1962 has a bolt receiver 1966. The stationary inner shaft 1952 is disposed through the outer rotating shaft 1954a and 1954b such that the stationary inner shaft 1952 is disposed at an angle (shown as 90 degrees in this implementation) where the plurality of slidable rods 1970 are also disposed at a similar angle.

The stationary inner shaft 1952 bend has an inner corner I and an outer corner O. It is appreciated that the stationary inner shaft 1952 and plurality of slidable rods 1970 may be machined to be disposed through and bent at any suitable angle. The plurality of slidable rods 1970 are slidably inserted circumferentially within the walls of the outer rotating shaft 1954a and 1954b, such that the slidable rods nearest inner corner I are fully inserted into the walls of the outer rotating shaft 1954a and 1954b, and the slidable rods nearest the outer corner O are at least partially inserted into the walls of the outer rotating shaft 1954a and 1954b. The amount of insertion of the slidable rods inserted between inner corner I and outer corner O is gradually increased or decreased as the outer rotating tube 1954a and 1954b rotate. As the outer rotating shaft 1954a and 1954b rotate, the insertion of the slidable rods rotating in the direction of inner corner I to outer corner O is decreased. Likewise, as the outer rotating tube 1954a and 1954b rotate, the insertion of the slidable rods rotating in the direction of outer corner O to inner corner I is increased.

In this implementation, as the output shaft 1956 rotates clockwise as does outer rotating tube 1954a and the plurality of slidable rods 1970. As the plurality of slidable rods 1970 rotate clockwise they rotate the outer rotating shaft 1954b clockwise and the nut receiver 1964. Likewise, rotation of the output shaft 1956 in a counterclockwise direction also rotates the outer rotating shaft 1954a in a same direction, along with the plurality of slidable rods 1970. As the plurality of slidable rods 1970 rotate counterclockwise the outer rotating tube 1954b can rotate in a counterclockwise direction, along with the nut receiver 1964. As the example device 1900 is powered by suitable means, the nut receiver 1964 applies torque to a target fastener (not shown), while the distal end 1966 of the spring 1962 applies a counter torque to a target component, such as 2126 in FIGURE 21C. The angle in the diagram is shown as 90 degrees, but it is contemplated that it may be configured to any suitable angle.

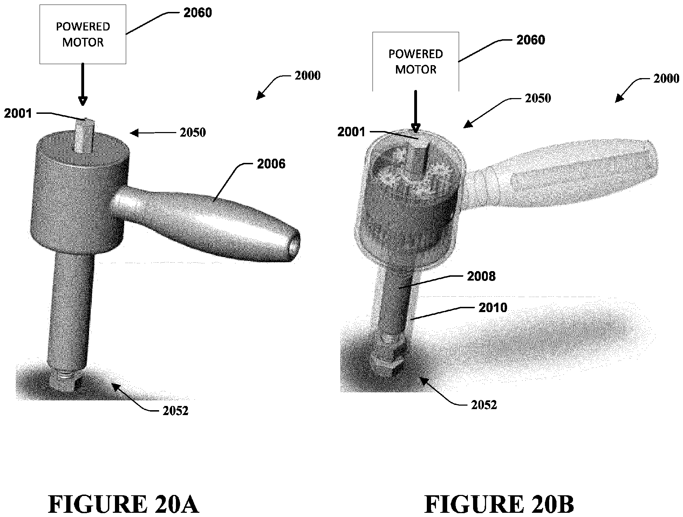

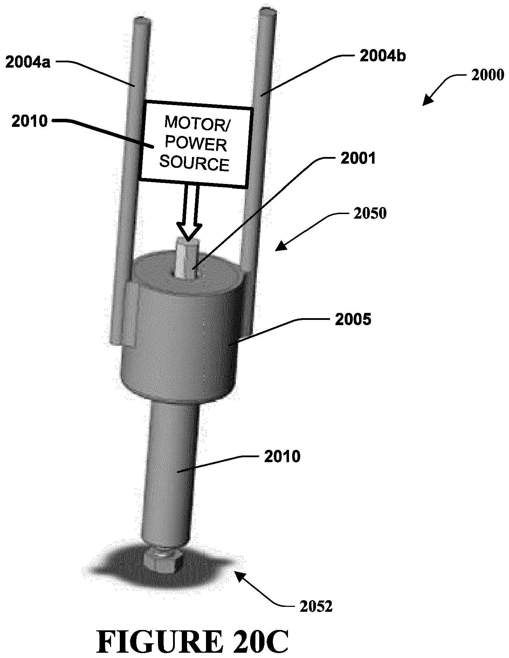

FIGURES 20A, 20B, and 20C are component diagrams that illustrate another alternate implementation of a device for applying torque and counter-torque. In this implementation, an example driver 2000 is adapted to receive a non-manual power source 2060 (e.g., a motor powered by a remote power source, such as a corded or cordless drill/driver). As illustrated in FIGURES 20A and 20B an example driver 2000 has a proximal or first end 2050 and a distal or second end 2052. On the proximal end 2050 there is a male hex shaft 2001. The male hex shaft 2001 can be fitted into a non-manual power source (such as a cordless drill, not shown). In this implementation, when torque is applied by a non-manual power source to the example driver 2000 through the male hex shaft 2001, a user can secure handle 2006 and apply counter torque by holding handle 2006 stationary. In the example shown, the driver 2000, optionally comprises a stationary inner shaft 2008 and an outer rotating tube 2010, this allows for example nut and bolt-type fastening at the distal end 2052 (described in detail below with FIGURES 21).

In another implementation as illustrated in FIGURE 20C, an example driver 2000 has a proximal or first end 2050 and a distal or second end 2052. On the proximal end 2050 there is a shaft adapter 2001 (e.g., male hex shaft, or other similar type of adapter) and posts 2004a and 2004b. The shaft adapter 2001 can be fitted into a non-manual power source 2010 (e.g., a cordless drill, portable motor, pneumatic power source, etc.). In some implementations, the power source 2010 can be disposed in the housing (e.g., 150 of FIGURE 1) of the device 2000. In other implementations, the power source 2010 can be portable, and have a coupler (e.g., collar) configured to couple with the shaft adapter 2001, Further, in some implementations, the coupler may have holes configured, to receive posts 2004a and 2004b. The posts 2004a and 2004b are fixedly attached to the outer housing 2005, such that when torque is applied to the example driver 2000 through the male hex shaft 2001, the posts 2004a and 2004b received in the collar of the cordless drill apply counter torque. The example driver 2000, optionally comprises a stationary inner shaft 2008 (not shown) and an outer rotating tube 2010, this allows for example nut and bolt-type fastening at the distal end 2052 (described in detail below with FIGURES 21).

In FIGURES 21A, 21B, 21C, and 21D are component diagrams illustrating an example driver 2100 that contains a torque inversion mechanism 2110, which inverts or transmits the torque applied to the inner rotating input shaft 2102 to an outer rotating tube 2104. As shown in FIGURE 21A, torque is manually applied through handle 2106 to an inner shaft 2102, the torque is transferred to the outer rotating tube 2104, such that outer rotating tube 2104 is the only rotating component, while the handle 2106 and housing 2108 are not rotating. As described in the implementations with FIGURE 19E, 19F, 19G, and 19H, the example driver 2100 may have an angled outer rotating tube 2104 to fasten a target in a tight or hard to reach situation. As described in the implementations with FIGURE 20A, 20B, and 20C, the example driver 2100 may be powered by a non-manual power source. In this implementation, the torque inversion mechanism 2110 can comprise a planetary gear arrangement. In some implementations, the planetary gear arrangement functions as the torque conversion mechanism (802 of FIGURES 8A-H) and the torque inversion mechanism 2110. FIGURE 21B illustrates an implementation of the torque inversion mechanism 2110, comprising the input shaft 2102, a sun gear 2112, planetary gears 2114 (four planetary gears are shown, but any suitable number may be used), and an inverting ring gear 2116. In this implementation, the input shaft 2102 is fixedly attached to the sun gear 2112, the sun gear 2112 meshedly engages the planetary gears 2114, the planetary gears 2114 meshedly engage the inverting ring gear 2116, and the inverting ring gear 2116 is free to rotate. In this implementation, this results in planetary gears 2114 orbiting the sun gear 2114, providing rotation to the inverting ring gear 2116. In this implementation, a torque limiter such as described in FIGURE 17A, 17B, and 17C is used. The inverting ring gear 2116 has a pin 2118 that is operably connected into a notch 2120 of the outer rotating tube 2104, such that when the inverting ring gear 2116 rotates it causes the outer rotating tube 2104 to rotate. If the counter torque exceeds the torque capacity of the torque limiter, then the pin 2118 rotates out of the notch 2120 and the outer rotating tube 2104 cannot apply the tightening torque. Optionally, no torque limiter may be present to allow the inverting ring gear 2116 to be directly connected to the outer rotating tube 2104.

FIGURES 21C and 21D are bottom views of the example driver 2100. As shown in FIGURE 21C, when the outer rotating tube 2104 is rotating, this allows for example nut and bolt-type fastening. In this implementation, a stationary inner shaft 2122 is disposed within the outer rotating tube 2104. The stationary inner shaft 2122 engages a bolt 2126 securing it with a male-female connection. As the stationary inner shaft 2122 secures the bolt 2126 and prevents it from rotating, the outer rotating tube 2104 rotates a nut 2124 threading it around the bolt 2126. As shown in FIGURE 21D, the outer rotating tube 2104 has a hexagonal receiver 2128 to engage the nut 2124 (not shown) and cause it to rotate. The outer rotating tube 2104 sits on the nut 2126 during operation to apply torque. It may be appreciated, that the outer rotating tube 2104 may have a receiver-shape to engage any suitable fasteners.

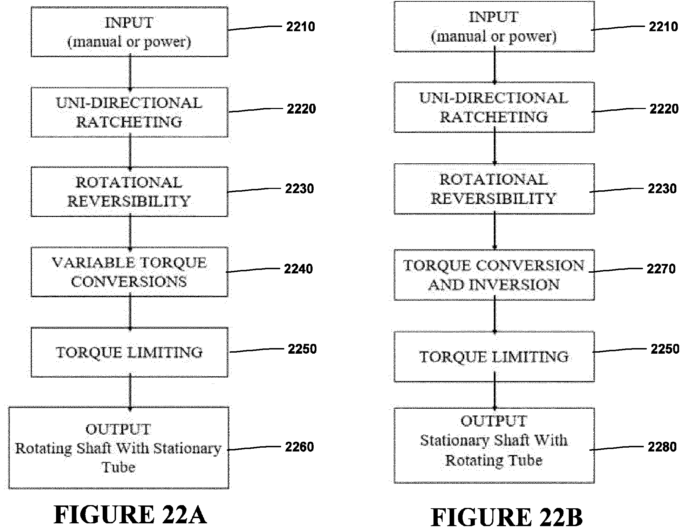

In FIGURES 22A and 22B are flowcharts illustrating an example implementation of combinations of one or more portions of the one or more systems described herein. As shown in FIGURE 22A, an input power source 2210 is applied to the input shaft of an example device. As described above (FIGURES 7A, 7B, 7C, and 10), as torque is applied from the input power source 2210 to the input shaft the uni-directionally ratcheting 2220 ensure uni-directional rotation, even if a reversal during, for example, manual operation occurs. As described above (FIGURES 4A, 4B, 11A, 11B, 11C, and 11D) the direction of torque applied to the input shaft may be reversed 2230 through a switch/button. As described above (FIGURES 8A, 8B, 8C, 8D, 8E, 8F, 8G, and 8H) the torque applied to the input shaft may be converted 2240 (such as 1:1, 1:2, 1:3, 1:4 etc.). As described above (FIGURES 9, 17A, 17B, 17C, 18A, 18B, 18C, 18D, 18E, 18F, and 18G), the torque applied to the input shaft may be limited 2250. As described above (FIGURES 6, 12, 13, 14, 15A, 15B, 16A, 16B, 19A, 19B, 19C, and 19D) the torque applied to the input shaft is transferred to the output, which is a rotating shaft, with a stationary tube/stationary housing 2260.

As shown in FIGURE 22B, an input power source 2210 is applied to an input shaft of the example device. As described above (FIGURES 7A, 7B, 7C, and 10), as torque is applied from the input power source 2210 to the input shaft the uni-directionally ratcheting 2220 ensure uni-directional rotation, even if a reversal during, for example, manual operation occurs. As described above (FIGURES 4A, 4B, 11A, 11B, 11C, and 11D) the direction of torque applied to the input shaft may be reversed 2230 through a switch/button. As described above (FIGURES 8A, 8B, 8C, 8D, 8E, 8F, 8G, and 8H) the torque applied to the input shaft may be converted 2270 (such as 1:1, 1:2, 1:3, 1:4 etc.) and torque applied to input shaft is transferred/inverted 2270 to an outer tube. As described above (FIGURES 9, 17A, 17B, 17C, 18A, 18B, 18C, 18D, 18E, 18F, and 18G), the torque applied to the input shaft may be limited 2250). As described above (FIGURES 19E, 19F, 19G, 19H, 20A, 20B, 20C, 21A, 21B, 21C, and 21D) the torque applied to the input shaft is transferred to the rotating tube, with a stationary inner shaft 2280.

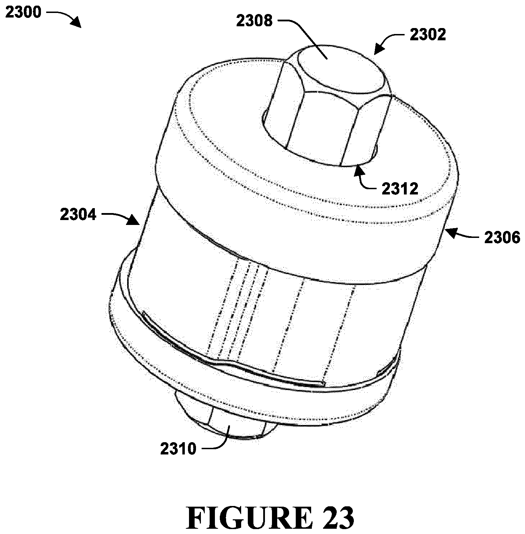

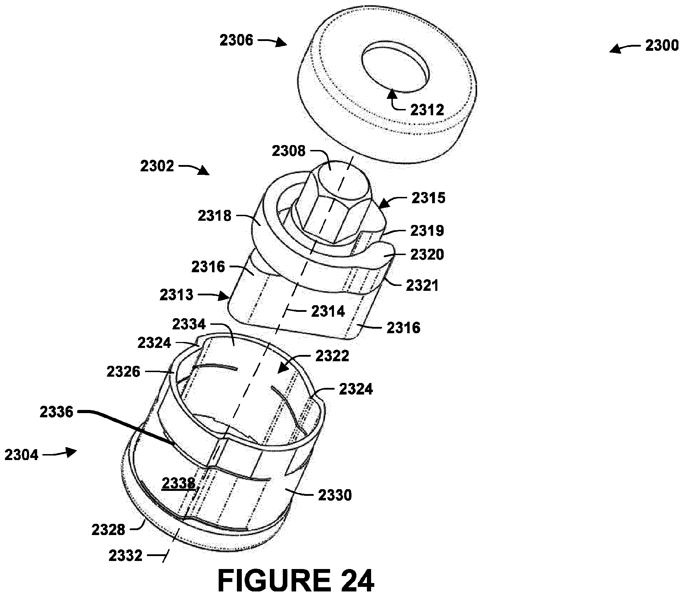

FIGURES 23 and 24 are component diagrams illustrating an example implementation of a torque limiter module 2300. The torque limiter module 2300 can be configured to provide a predetermined output of torque, which can be limited/set to a desired/preset torque value. As an example, a torque limiter module 2300 can be designed and manufactured to provide a predetermined torque limit, also referred to as torque level. In some implementations, the torque limiter module 2300 can be configured to provide a predetermined limit of torque output (e.g., optionally pre-selected), which can be preselected at different desired torque values. In one example, the torque limiter module can be configured to provide a predetermined limit of torque output that can be preselected mechanically at different desired torque values such as, for example, mechanically selecting a torque limit by engaging designated mechanical features. In another example, the torque limiter module can be configured to provide a predetermined limit of torque output that can be preselected electronically at different desired torque values such as, for example, electronically selecting a torque limit using a toggle switch.

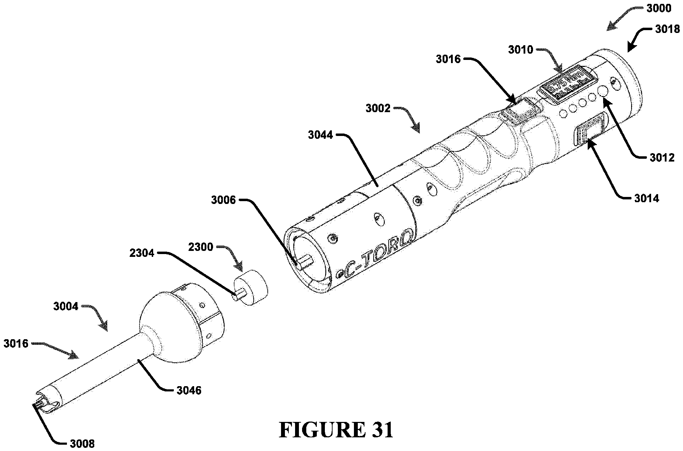

Additionally, in some implementations, a plurality of torque limiter modules (e.g., 2300) can be prepared, each having a different pre-selected torque limit value. In these implementations, a desired (e.g., for a target operation) torque limit can be selected for a counter-torque device of this disclosure (e.g., 100 of FIGURES 1A-1G; or 200 of FIGURES 2A-2G, 6, or 3000 of FIGURES 31, 32, 33) by selecting the torque limiter modules (e.g., 2300) that corresponds to the desired torque limit. In these implementations, for example, the selected torque limited module, having the desired torque limit, can be installed in the counter-torque device and used for the target operation. That is, for example, instead of having a selectable or adjustable torque limit for the counter-torque device, the torque limit can be changed by changing out the torque limiter modules (e.g., 2300).

The torque limiter module 2300, as illustrated in FIGURES 23 and 24, can be configured for use in a device of this disclosure (e.g., 100 of FIGURES 1A-1G; or 200 of FIGURES 2A-2G, 6, or 3000 of FIGURES 31, 32, 33) to apply torque to a target component in a first direction while applying counter-torque in a second direction. In one implementation, the torque limiter module 2300 is configured for use in a mechanical device that is manually driven (e.g., 100 of FIGURES 1A-1G) for applying torque and counter-torque. In another implementation, the torque limiter module 2300 is configured for use in an electronic device that is power driven (e.g., 200 of FIGURE 6) for applying torque and counter-torque. For example, the use of an electro-mechanical torque limiter is anticipated for devices using an electric motor as a power source. In those implementations the current draw by the electrical motor can be monitored, and compared to identified pre-set values that corresponds to torque applied at the monitored current draw. In these implementations, the desired torque limit can be selected, and the motor can be shut off at the selected torque value corresponding to the current draw during use. That is, for example, a controller can turn off the motor when the current draw has reached a value that correspond to a predetermined torque level.

As illustrated in FIGURE 23, as described above, the torque limiter module 2300 can be a separate, individual module. Additionally, in some implementations, the torque limiter module 2300 can be disposable. It is to be appreciated that an individual, disposable torque limiter module 2300 can provide several benefits. For example, different models of individual torque limiter modules 2300 that are disposable can be provided with each model specifically configured to provide a predetermined amount of applied torque. Thus, different individual, disposable torque limiter modules 2300 can be designed and manufactured to provide different predetermined torque limits (or torque levels), and a user can select a specific individual, disposable torque limiter module 2300 for use in a device for applying torque and counter-torque based on need, such as for a target operation (e.g., inserting a pedicle screw), or applying the recommended torque level of the device. In some implementations, the torque limiter module 2300 can be reusable. As an example, the torque limiter module 2300 may be reusable where any of its respective openings, or holes 2312, can be appropriately sealed for sterilization, or the device is configured to be sterilized. Further, the use of a selectively replaceable, and disposable, torque limiter module 2300 eliminates the need to re-calibrate the torque limiting module 2300 before use, as a new, and already calibrated module can be used.

The torque limiter module 2300 can comprise an input component 2302 and an output component 2304, as shown in FIGURES 23 and 24. The input component 2302 can comprise an input connection (e.g., shaped shaft or nut, configured to receive a complementary shaft or similar), such as a drive shaft 2308 that drives the input component 2302 to rotate by applying torque to the input component 2302. The drive shaft 2308 is operably engaged with other components that apply torque to the drive shaft 2308 causing it to rotate. The output component 2304 can include an output connection, such as an output shaft 2310 for operably engaging with other components and configured to transfer the torque applied to the output shaft 2310 to other components that are operably attached to the output shaft 2310. The torque limiter module 2300 can be included in a device for applying torque and counter-torque and subsequently arranged in between other components such as, for example: (i) a distal tip and power pack; (ii) below torque multiplier for high torque capacity; or (iii) above torque multiplier for low torque capacity. As an example, the torque limiter module 2300 may be a separate, individual module that is disposable and/or reusable, and comprises an input component 2302 that is driven to rotate by torque applied by a drive shaft 2308 operably attached (or rigidly connected) thereto, and an output component 2304 that is fixedly attached (or rigidly connected) to an output shaft 2310, such that torque applied to the output shaft 2310 is transferred to other components that are fixedly attached thereto.

It should be noted, that in some implementations, the input component 2302 and output component 2304 can be reversed in the module 2300 (e.g., and in disposition between the power source portion 3002 and distal tip engagement portion 3004). That is, for example, the input connection 2308 of the input component 2302 can become an output drive shaft, and the output shaft 2310 of the output component 2304 can become an input connection - such as might be if the module 2300 is reversed.

As an illustrative example, as shown in FIGURE 31, the torque limiter module 2300 can comprise a selectably removable component that is operably disposed in an example torque/counter-torque device 3000. In this example, the torque limiter module 2300 can be disposed between a proximal power source portion 3002 and a distal tip engagement portion 3004. In this example, the power source portion 3002 (e.g., comprising an electrical supply, motor, gearing, controller, selector switches, etc.) can provide rotational power at a distal powered shaft 3006, which selectably engages with the input component 2302. Further, the distal tip engagement portion 3004 selectably engages with the output component 2304, which provides the rotational power to the distal tip engagement portion 3004. The distal tip engagement portion 3004 provides torque to an engagement tip 3008, such as through an internal drive shaft, which operably engages with a target fastener, for example,

In some implementations, as illustrated in FIGURE 24, the torque limiter module 2300 can comprise an input component 2302, an output component 2304, and a lid 2306. The lid 2306 is an optional component and may include an opening 2312 having a size and shape configured to accommodate a drive shaft 2308 extending therethrough. It is to be appreciated the lid 2306 can be used to close the torque limiter module 2300 into a single unit or module, as illustrated in FIGURE 23. The lid 2306 may also render the torque limiter module 2300 reusable by covering and sealing any openings to mitigate fluid migration inside the torque limiter module 2300. In one non-limiting example, the torque limiter module 2300 comprises an input component 2302, an output component 2304, and a lid 2306 that, when assembled, closes the torque limiter module 2300 into a single unit with a drive shaft 2308 extending through an opening 2312 in the lid 2306. The drive shaft 2308 may fit snuggly in the opening 2312 providing a sealing engagement such that openings of the assembled torque limiter module 2300 are sufficiently sealed to mitigate fluid migration into the interior of the torque limiter module 2300.

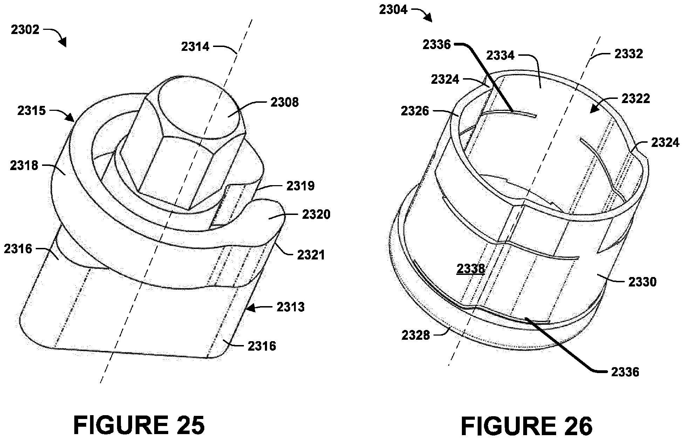

The torque limiter module 2300, as illustrated in FIGURES 24 and 25, can be configured to provide a predetermined limit of torque output (e.g., optionally pre-selected), which can be preselected at different desired torque values. For example, the torque limit of a torque limiter module 2300 can be set at 0.5 Nm and can also be set at 15 Nm (e.g., and values in between). In some implementations, the torque limit for each torque limiter module 2300 can be predetermined based on the manufacturing dimensions of the input component 2302 and the output component 2304. As an example, small torque limiter modules 2300 can be provided that have different manufacturing dimensions for the input and the output components 2302 and 2304 for the purpose of providing different torque limits that can be pre-selected mechanically. In one example implementation, one of these torque limiter modules can be selected that is operable for the preselected amount of torque (e.g., the specified or desired torque value/level) and subsequently included in a mechanical device that is driven (e.g., manually or non-manually powered) to apply torque and counter-torque.

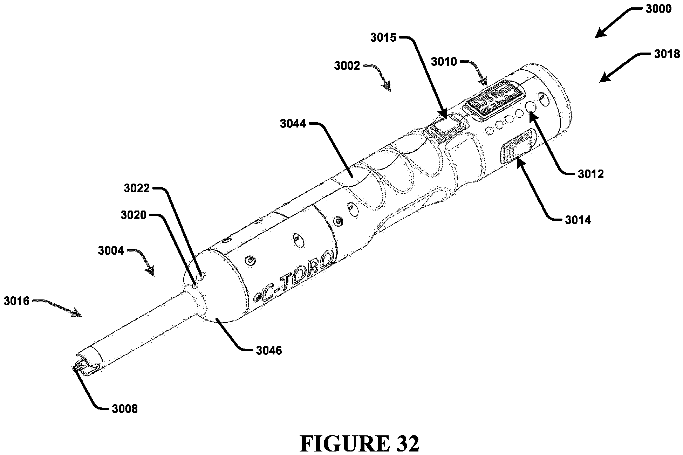

Alternatively, or additionally, as another example a torque limiter module 2300 can be configured to provide different torque limits that can be selected electronically, for example, by activating a selection icon, button, dial, or switch, such as a toggle switch or slide switch (e.g., 256 of FIGURE 2, or 3014 of FIGURES 31, 32) arranged on the device for applying torque and counter-torque (e.g., disposed on the housing 220 of the device 200 in FIGURE 2, or 3044 of FIGURES 31, 32).

As an example, a selection button or switch can be used to select the rotation direction of the applied torque, and/or change an amount of torque/rotation applied (e.g., as a torque limit). As an example, the device for applying torque and counter-torque (e.g., 200 of FIGURE 2, 3044 of FIGURES 31, 32) can include a slide switch (e.g., 3015 of FIGURES 31, 32) that is selectably activated by translating the switch in a first direction and a second direction (e.g., up or down; left or right) to select the rotation direction of the applied torque, and/or change the amount of torque/rotation applied. In one example implementation, a torque limiter module that provides for electronic selection of the amount of torque (e.g., the desired torque value/level) can be included in an electronic device that is power driven to apply torque and counter-torque. It is to be appreciated the power can be provided by a power source that is powered manually, by electric motor, pneumatic drive, hydraulic drive, or any other suitable means.

The torque limiter module 2300, as illustrated in FIGURES 23 and 24, can be included in a mechanical device or an electronic device for applying torque and counter-torque. In some implementations, the torque limiter module 2300 can be positioned or seated in a low torque region, for example, proximal to a planetary gear arrangement (e.g., 808, 814 of FIGURES 8A-8H). In other implementations, the torque limiter module 2300 can be positioned or seated in a high torque region, for example, distal to a planetary gear arrangement (e.g., 808, 814 of FIGURES 8A-8H). It is to be appreciated that the lid 2306 is an optional component of any torque limiter module 2300 and its inclusion or absence may not affect the provision of the predetermined torque level.

As illustrated in FIGURES 24 and 25, the input component 2302 can comprise a first section 2313 and a second section 2315. The first section can comprise a plurality of tips 2316 and the second section 2315 can comprise a radial leaf spring 2318. The drive shaft 2308 may extend through the first section 2313 and second section 2315 of the input component 2302 in an axial direction to define an axis of rotation 2314 of the input component 2302. The input component 2302 can be driven such as, for example, rotated in a clockwise or counterclockwise direction, with torque applied by the drive shaft 2308. The axis of rotation 2314 extends in the axial direction. In some implementations, as illustrated in FIGURE 25, the axis of rotation 2314 may be disposed in the center of the input component 2302.

The first section 2313 can comprise a plurality of tips 2316, sometimes also referred to as noses. Each tip 2316 of the plurality of tips 2316 may extend radially outward from the center of the first section 2313 in a direction transverse to the axial direction and axis of rotation 2314. In some implementations, the length or extension of the respective tips 2316 can be equal or may be different for each. Each tip 2316 can have a substantially rounded end configured to selectably engage outer walls 2330 (or perimeter walls) of the output component 2304 and, more specifically, a protrusion 2324 extending from the outer walls 2330 inside the cavity 2322, as described in more detail below. In some implementations, as illustrated in FIGURES 24 and 25, the input component 2302 can comprises three tips 2316. In other implementations, the input component 2302 can comprise one, two, four or more tips 2316. The exact number of tips 2316 disposed on the input component 2302 can vary but is generally selected to correspond to, and preferably correspond to, the number of protrusions 2324 disposed on the output component 2304, as described in detail below.

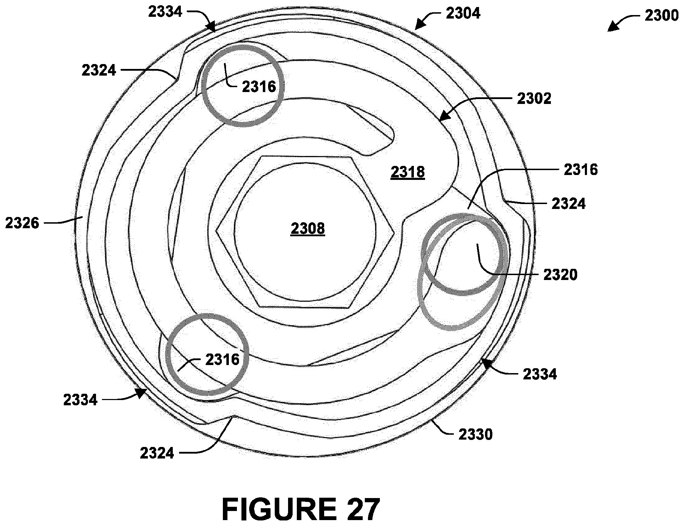

The input component 2302, as illustrated in FIGURES 24 and 25, can comprise a radial leaf spring 2318 extending around at least a portion of the axis of rotation 2314 such that the radial leaf spring 2318 surrounds at least a portion of the axis of rotation 2314. In some implementations, the radial leaf spring 2318 can be nearly circumferential and surround most of the axis of rotation 2314. As illustrated in FIGURE 27, the radial leaf spring 2318 can have a first end 2319 fixedly engaged with the drive shaft 2308 or a drive shaft covering (e.g., sleeve or wrap over the drive shaft) and a second end 2321 that terminates in a tip 2320 that is free-floating and deflectable. In some implementations, as illustrated in FIGURES 25 and 27, the radial leaf spring 2318 can have a thicker portion at its tip 2320 (e.g., an enlarged tip 2320), sometimes also referred to as a clicker, for the purpose of making an audible nose (e.g., producing an audible "click") and producing a tactile feel to indicate a preselected amount of torque (the desired torque value/level) has been reached as explained below.

The output component 2304 functions as a container or housing and comprises an open first end 2326, a closed second end 2328 disposed opposite the open first end 2326, and a hollow interior space, or cavity 2322, that is configured to receive the input component 2302 therein. As an example, an assembled torque limiter module 2300 can comprise an input component 2302 disposed within the cavity 2322 of the output component 2304. The output component 2304 can further comprise an output shaft 2310 rigidly connected thereto, for example, to an outer surface (e.g., exterior surface) of the output component 2304. In some implementations, as illustrated in FIGURE 23, the output shaft 2310 can be rigidly connected to the closed second end 2328 of the output component 2304 and extend out from the closed second end 2328 in an axial direction to define an axis of rotation 2332 of the output component 2304. It is to be appreciated the output shaft 2310 is for operably engaging other components and can be configured to transfer the torque applied to the output shaft 2310 to other components that are fixedly attached to the output shaft 2310.

In one implementation, as illustrated in FIGURES 24 and 26, the output component 2304 can comprise an open first end 2326 (e.g., an open top), a closed second end 2328 disposed opposite the first end 2326 (e.g., a closed bottom), and outer walls 2330 (or perimeter walls) extending between the open first end 2326 and closed second end 2328 and defining the hollow interior cavity 2322 having dimensions configured to operably accommodate the input component 2302 within the cavity 2322, such that the first section 2313 comprising the plurality of tips 2316 is disposed proximate the closed second end 2328 of the output component 2304 and the second section 2315 comprising the radial leaf spring 2318 is disposed proximate the open first end 2326. Alternately, in some implementations, the radial leaf spring 2318 can be disposed beneath the first section 2313, towards the closed second end 2318.

As illustrated in FIGURES 24, 26 and 27, the output component 2304 can comprise a plurality of protrusions 2324 disposed on an inside wall of the cavity 2322 of the output component 2304, each of which extends from the inside of the outer walls 2330 to the inside of the cavity 2322. In some implementations, the protrusions 2324 can extend radially inward toward a center of the output component 2304 in a direction transverse to the axial direction and the axis of rotation 2332. The plurality of protrusions 2324 comprises at least two protrusions. As an example, the output component 2304 can comprise four protrusions 2324, each of which extends from the outer walls 2330 inside the cavity 2322. In one implementation, as illustrated in FIGURES 24, 26 and 27, the output component 2304 can comprise three protrusions 2324, each of which extends from the outer walls 2330 inside the cavity 2322, radially inward toward the center of the output component 2304 (and of the cavity 2322) in a direction transverse to the axial direction and axis of rotation 2332.

In some implementations, as illustrated in FIGURES 24 and 26, the outer wall 2330 of the output component 2304 can comprise one or more relief slits 2336. For example, the relief slits 2336 can comprise cuts or elongated openings that run around a portion of the periphery of the wall. In this implementation, the location of the slit(s) 2336 aligns with the location of the respective protrusions 2324. That is, for example, at each protrusion 2324 the slit 2336 can be generally, centrally aligned. As such, if the output component wall 2330 comprise one protrusion, one or more slits may be positioned at that protrusion, for example. Further, if two protrusions 2324 (e.g., three, four, etc.) are disposed in the wall 2330, each of the two (*e.g., three, four, etc.) protrusions 2324 may have one or more slits 2336 disposed in alignment. In this example, the respective slits 2336 operably provide for relief for deflection of the wall 2330, at the protrusion 2324, such as when a tip/nose 2316 of the input component 2303 rotates past the protrusion 2324. For example, as illustrated, a pair of spaced apart slits 2336 are disposed at respective protrusions 2324. In this example, when the tip/nose 2316 rotates past the protrusion 2324 the middle section 2338 of wall 2330 can deflect outward, as the respective slits 2336 provide relief to release that section 2338. That is, for example, the size, location, and number of slits can help determine the torque limit (e.g., along with other factors) by managing an amount of deflection provided to the wall 2330 at the protrusion 2324.

As illustrated in FIGURES 24, 26 and 27, the output component 2304 can comprise a plurality of recesses 2334, or grooves, disposed in the inside wall the cavity 2322 of the output component 2304. Each of the plurality of recesses 2334 can be formed on the inside of the outer walls 2330 inside the cavity 2322. In some implementations, the recesses 2334 formed in the outer walls 2330 can extend radially outward, away from the center of the output component 2304 and in a direction transverse to the axial direction and axis of rotation 2332. In some implementations, the recesses 2334 can have a convex shape and may be curved or bowed outward, in a direction extending away from the center of the output component 2304 and in a direction transverse to the axial direction and axis of rotation 2332. In other implementations, the recesses 2334 can be formed by portions of the outer walls 2330 that are set back (e.g., recessed) relative to other portions of the outer walls 2330 in a direction extending radially outward and away from the center of the output component 2304, and in a direction transverse to the axial direction and axis of rotation 2332. The plurality of recesses 2334 comprises at least two recesses. In one implementation, as illustrated in FIGURE 27, the output component 2304 can comprise three recesses 2334, each of which is formed in the outer walls 2330 inside the cavity 2322 and extends radially outward and away from the center of the output component 2304, and in a direction transverse to the axial direction and axis of rotation 2332.

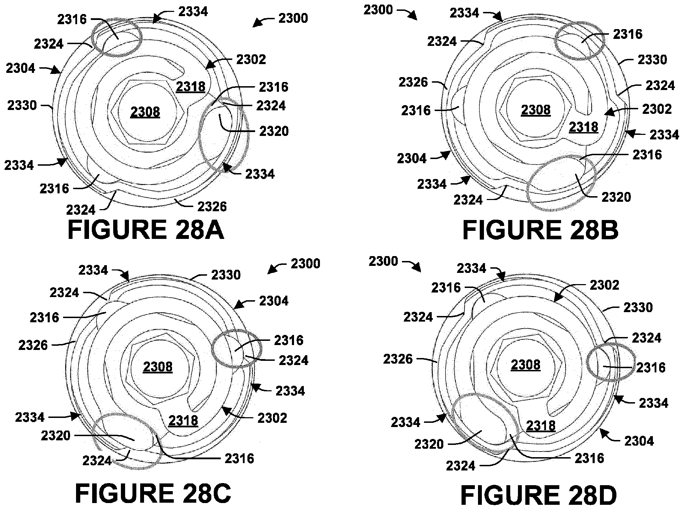

In some implementations, as illustrated in FIGURE 27, varying the thickness of the outer walls 2330 in a radial direction (e.g., in a direction transverse to the axial direction and axis of rotation 2332) can provide the plurality of protrusions 2324 and a plurality of recesses 2334. As an example, as illustrated in FIGURES 26 and 27, each protrusion 2324 can be formed by a thick portion of the outer walls 2330 that is disposed in between (e.g., sandwiched between) recesses 2334, or grooves, defined by (or formed in) thin portions of the outer walls 2330. In some implementations, as illustrated in FIGURES 28A through 28D, each protrusion 2324 may be formed by one or more segments of outer walls 2330 that progressively increase in thickness (e.g., in a radial direction) moving in one direction along the outer walls 2330 (e.g., along the perimeter). In one implementation, as illustrated in FIGURES 26 and 27, each protrusion 2324 may progressively increase in thickness moving in one direction (e.g., a rotational direction) around the outer walls 2330, for example, around the circumference in a clockwise direction. The protrusions 2324 do not need to be symmetrical. As an example, the protrusion 2324 may include a gradual incline for the purpose of slowly building torque, a peak to provide max torque, and a steep decline for the purpose of rapidly releasing torque.

The number of recesses 2334 can be selected to match the number of protrusions 2324 of the output component 2304. Additionally, the number of protrusions 2324 can be selected to match the number of tips 2316. In one implementation, as illustrated in FIGURE 27, the output component 2304 can comprise three recesses 2334 formed in the outer walls 2330 inside the cavity 2322, each of which is disposed in between (or sandwiched between) three protrusions 2324 such that each recess 2334 is sandwiched between two protrusions 2324, and the input component 2302 can comprise three tips 2316. It is conceivable some implementations do not include the same number of protrusions 2324 and tips 2316.

The exact number of protrusions 2324 can vary but is generally selected to match the number of tips 2316, or noses, of the first section 2313 of the input component 2302. The protrusions 2324 can be of a size and shape configured to engage the tips 2316 of the input component 2302 in a radial plane, such as when the input component 2302 is rotated in a clockwise or counterclockwise direction by torque applied by the drive shaft 2308. It is to be appreciated from this configuration that any impact force from snapping through at the torque limit is exerted (applied) in the radial direction and not the axial direction. It is to be appreciated that the torque limiter module 2300 can comprise protrusions 2324 and tips 2316 of different sizes to provide different predetermined torque levels, which may be optionally pre-selected. In this manner, the torque limiter module 2300 can be configured to provide a predetermined limit of torque output (e.g., optionally pre-selected), which can be preselected at different desired torque values.

It is to be appreciated that a high level of interference between the input component 2302 and the output component 2304 and, thus, predetermined torque limit, occurs when the tip 2316, or nose, slides past the protrusion 2324. For example, the thin walls (e.g., or merely a wall section between slits) of the output component may flex outward as the tips, or noses, of the input component rotate past respective complementary protrusions disposed in the output component indicative of when the torque limit of the torque limiter module 2300 has been reached.

A torque limiter module 2300 and, in particular, a mechanical torque limiter module functions according to interaction between the input component 2302 and the output component 2304. As an example, the input component 2302 can be inserted into cavity 2322 of the output component 2304 with the first section 2313 of the input component 2302 operably disposed within the cavity 2322 of the output component 2304 to provide for engagement and interference between the tips 2316 of the input component 2302 and the protrusions 2324 of the output component 2304. In one implementation, as illustrated in FIGURES 24 and 27, the input component 2302 can comprise a first section 2313 comprising three tips 2316 configured to engage three protrusions 2324 disposed inside the cavity 2322 of the output component 2304 such as, for example, by driving (e.g., rotating) the input component 2302 which is operably disposed within the cavity 2322 of the output component 2304.

As an example, the three tips 2316 of the first section 2313 can be configured to engage the three protrusions 2324 of the output component 2304 to transfer the torque to the output component 2304, as long as the torque does not exceed the predetermined torque limit. In one implementation, as illustrated in FIGURE 27, the three protrusions 2324 are disposed inside the cavity and configured to engage the three tips 2316 of the input component 2302 at a position where a highest amount of interference occurs between the input component 2302 and output component 2304. For example, as the tips 2316 engage the protrusions 2324 during applied torque, the rotations can be transferred to the output component 2304, while the thin wall sections of the output component 2304 are pushed outward and the tips 2316 on input component 2302 are pushed inward. In some implementations, as described above, the output component 2304 further comprises relief slits 2336 at the protrusions 2324 to allow at least a portion of the outer walls 2330 to deform outward. It is to be appreciated that the amount of predetermined interference, which is equivalent to the amount of deformation, is directly related to the torque limit desired.

The second section 2315 of the input component 2302 can comprise a radial leaf spring 2318 that has a thicker portion at its tip 2320 (e.g., an enlarged tip 2320), sometimes also referred to as a clicker, for the purpose of making an audible nose (e.g., producing an audible "click") and producing a tactile feel to indicate a preselected amount of torque (the desired torque value/level) has been reached. As an example, when the radial leaf spring 2318 disengages from the protrusion 2324 and moves past the protrusion 2324 an audible sound is produced, such as a "click", and a tactile feel provided indicating when the desired torque level has been reached. It is conceivable that the tips 2320 of the radial leaf spring 2318 may further comprise rollers or bearing pins to mitigate sliding friction between the input component 2302 and output component 2304.

The input and output components 2302 and 2304 of the torque limiter module 2300 are not limited to any particular configuration and may assume a variety of different sizes, shapes, and dimensions. In one implementation, as illustrated in FIGURE 23, the torque limiter module 2300 comprises an input component 2302 and an output component 2304 that each have a male hex-shape or male-hex configuration. However, the input and output components 2302, 2304 are not limited to any particular shapes and/or dimensions and can have other configurations.

FIGURES 28A through 28D illustrate the process of torque transfer in a torque limiter module 2300. In particular, FIGURES 28A through 28D illustrate several stages of torque transfer associated with driving (or rotating) the input component 2302 of the torque limiter module 2300. As an example, FIGURE 28A shows a torque limiter module 2300 in a no-torque position or torque-free position. In the no-torque position, all three tips 2316 (one is circled in red) are disposed in a recess 2334 of the output component 2304. There is little to no (i.e., negligible) engagement and interference between the tips 2316 and the protrusions 2324. In this condition, or torque transfer configuration, the tips 2316 are disposed in a low torque state. Additionally, the radial leaf spring 2318 and, in particular, the enlarged tip 2320 (shown in green) of the radial leaf spring 2318 is disposed in a recess 2334 of the output component 2304 and is also disposed in a low torque state.

As another example, FIGURE 28B shows a torque limiter module 2300 where the input component 2302 has been rotated (e.g., driven) in a clockwise direction. As the input component 2302 is rotated clockwise, the three tips 2316 of the first section 2313 rotate clockwise and begin to engage and apply force to the outer walls 2330 that define the protrusions 2324 inside the cavity 2322. In particular, the outer walls 2330 progressively increase in thickness along the direction of rotation, the clockwise direction, to form a protrusion 2324 that extends radially inward toward a center of the output component 2304 in a direction transverse to the axial direction and the axis of rotation 2332. Interference between the tips 2316 of the first section 2313 and the outer walls 2330 defining the protrusions causes rotation of the output component 2304. In this particular example, the torque transmitted determines how much force is applied by, or pressure is exerted by, the input component to the output component resulting from rotation of the input component. Further, the radial leaf spring of the clicker is also compressed due to interference between the tips of the input component and the walls defining the protrusions of the output component.

At FIGURE 28C, the three tips 2316 of the input component 2302 have reached a highest interference with the protrusions 2324 of the output component 2304. The three tips 2316 of the input component 2302 engage the protrusions 2324 of the output component 2304 and apply a highest amount of force (e.g., for the set torque limit) to, or exert highest pressure on, the protrusions 2324 disposed in the interior cavity 2322 of the output component 2304 providing for a highest level of transference of torque from the input component 2302 to the output component 2304. In some implementations, the force applied by, or exerted by, the input component to the perimeter wall of the output component can cause the perimeter wall to be pushed, or deflected, outward. It is to be appreciated that exceeding the highest torque value (e.g., pre-selected torque limit), may cause the input component to snap thru, or snap past, the protrusion, the output component will no longer rotate due to release of the force applied by the input component (e.g., disengagement of tips 2316 from protrusions 2324).

At FIGURE 28D, the input torque on the input component 2302 has exceeded the pre-selected torque limit of the torque limiter module. In this example, this results in the tips 2316 of the input component 2302 to snap past the respective protrusions 2324 of the output component 2304. Subsequently, the input component 2302 no longer applies rotation to the output component 2304. In some implementations, the bent leaf spring 2318 of the clicker 2320 may also snap thru and forcibly engage the perimeter wall 2330 of the output component 2304 creating an audible "click" and a tactile feel.

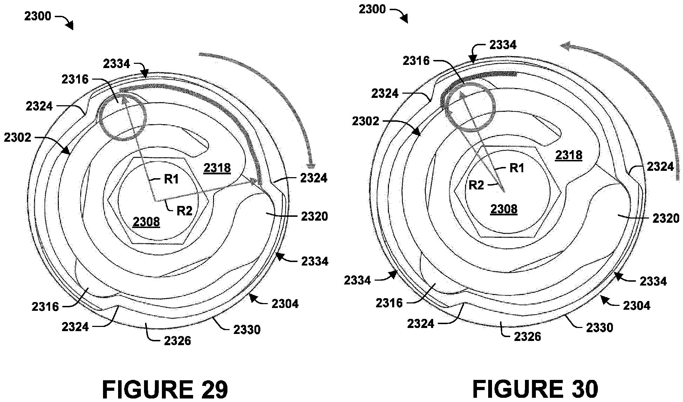

FIGURES 29 and 30 are component diagrams illustrating the direction of rotation of an input component 2302 relative to an output component 2304 in a torque limiter module 2300. For example, the input component 2302 may rotate in a clockwise direction relative to the output component 2304, as illustrated in FIGURE 29. Alternatively, the input component 2302 may rotate in a counterclockwise direction relative to the output component 2304, as illustrated in FIGURE 30. FIGURES 29 and 30 show the relationship between torque limit and the direction of rotation (e.g., clockwise direction or counterclockwise direction) of the input component 2302 relative to the output component 2304 in a torque limiter module 2300. For example, FIGURES 29 and 30 demonstrate that the torque limit cam be lower during a clockwise rotation of the input component 2302 than during counterclockwise rotation of the input component 2302. This can be particularly advantageous considering that a higher amount of torque is generally required for untightening (e.g., loosening) than for tightening, such as for a screw or nut.

As illustrated in FIGURE 29, the input component 2302 may rotate in a clockwise direction relative to the output component 2304. The clockwise rotation of the input component 2302 results in the clockwise rotation of the input component tips 2316 such that the tips 2316 are displaced along the perimeter of the input component 2302. As an example, clockwise rotation of the input component 2302 results in the tips being radially displaced inward within the cavity 2322 of the output component 2304. As illustrated in FIGURE 29, the input component 2302 and, more specifically, the tips 2316 of the input component may rotate in a clockwise direction relative to the protrusions 2324 of the output component 2304, and may engage the protrusions 2324 to transfer rotation to the output component 2304. In this example, the torque limit for the clockwise rotation will be less than the torque limit in a counterclockwise direction due to a gradual encroachment of the protrusion 2324 in the clockwise direction and a very sharp encroachment in the counter-clockwise direction. For example, the protrusion 2324 can be formed by the wall 2330 gradually sloping inward in a direction of a first rotational action to a sharp shoulder (the protrusion) that radiates outward. This gradual slope to the protrusions provides for the pre-set torque limit. That is, the pressure/interference between the tips 2316 and the protrusions 2324 is lower in the clockwise direction than the counter-clockwise direction, in these examples.

Alternatively, in another example implementation, as illustrated in FIGURE 30, the input component 2302 may rotate in a counterclockwise direction relative to the output component 2304. As described above, the tips 2316 engage the protrusions 2324 to transfer rotations force from the input component 2302 to the output component.

Figures 31 and 32 illustrate one example implementation of a torque/counter-torque device 3000, as described herein. This example is illustrative to show example embodiments of various systems, components, and devices that may be implemented in/with the device 3000, although these implementations are non-limiting examples of additional functionality and improved use for the device 3000. In one implementation, the example torque/counter-torque device 3000 can comprise a system that uses detected electrical use to provide feedback to a user, and/or to control the torque applied at the engagement tip 3008, as described above. In this implementation, the amount of current used (e.g., drawn, as in amps) can be detected during use, and correlated to an amount of torque being applied by the device 3000. That is, for example, empirical testing can evaluate the amount of torque applied for any given current load, and data indicative of this relationship (e.g., function) can be loaded into memory of a controller (e.g., control board) in the device 3000. Then, during use, a processor disposed on the controller can detect the current load and use the stored relationship to determine the amount of torque being applied. In these implementations, this information can be used to provide feedback to the user, and control the motor of the device 3000.

In one implementation, the device 3000 can comprise a display 3010 (e.g., LCD, or other functional display), which can be operably used to display use feedback to the user. For example, during use, the display 3010 can display the amount of torque being applied (e.g., instantaneous torque, such as in newton meters (Nm) or other appropriate units). Further, the display 3010 can display a torque limit set by the user. For example, the device 3000 can have user inputs (e.g., buttons, switches, dials, widget on a touch display, etc.), such as a toggle slider switch 3014, that can be used to select a torque limit; and/or the device 3000 can have a communications component (e.g., wired or wireless) that allows for a torque limit (e.g., and other data) to be loaded to the memory of the controller. Additionally, an array of indicators 3012 (e.g., LED lights) can be used to provide an indication of torque applied, such as by a number and color of lights displayed (e.g., from green to yellow to red lights, with green being low, yellow being medium, and red being high).

In some implementations, the device 3000 can comprise a power/direction user input 3015, such as a toggle selector switch (e.g., buttons, switches, dials, widget on a touch display, etc.) that may be used to control (e.g., alter) direction of rotation of the applied torque, between clockwise and counter-clockwise. Further, the power/direction user input 3015 can be configured to control an amount of power of and/or speed of the shaft rotation. That is, in some implementations, the power/direction user input 3015 can be coupled with the controller to control an amount of power provided to, and/or the speed of rotation of the motor. Thus, the speed of rotation of the shaft can be controlled. In some implementations, the direction of rotation and speed of rotation can be controlled by different use inputs (e.g., switches).

In some implementations, the device 3000 can comprise an illumination component 3020, such as a light, disposed at the distal end 3016 of the device 3000. During operation, the illumination component 3020 can be activated to illuminate a target operational area. Further, in some implementations, the 3000 can comprise an image sensor 3022, such as a camera, disposed at the distal end 3016 of the device 3000. In this implementation, during operation, the image sensor 3022 can be activated to generate images (e.g., photos, video) of the target operational area.

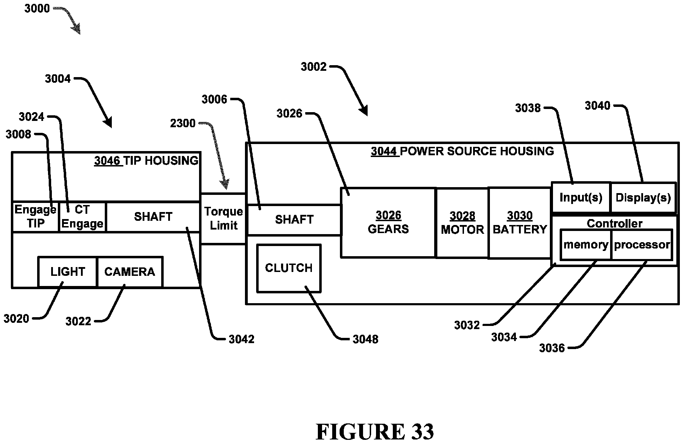

As illustrated in FIGURE 31, and schematically in FIGURE 33, the example device 3000 comprises a selectably separable power source portion 3002 and distal tip engagement portion 3004, having the torque limiter 2300 disposed therebetween. In some implementations, the selectably separable power source portion 3002 and distal tip engagement portion 3004 can be fixed together using conventional fasteners, latches, twist-lock engagement, and/or using magnets. Further, the power source portion 3002 can comprise housing 3044 that houses the distal powered tip 3006, described above, which is operably coupled with gearing 3026, such as a gear box comprising gears that provide for torque conversion, directional control, speed control, and more, as described above (e.g., FIGURES 7A-C, 8A-H, and 11A-D). Power can be provided to the gears by a motor 3028, which is powered by a battery 3030, such as a rechargeable battery, as described above.

As described herein, the power source portion 3002 can also comprise a controller 3032, which comprises memory 3034 and a processor 3036. The memory 3034 can store data and instructions used for various operations of the device 3000, and the processor can be used to process the data and instructions to perform the operations. As an example, the memory 3034 can store data indicative of use of the device, such as torque applied during operations, which can be downloaded for later processing and viewing. Additionally, one or more inputs 3038 (e.g., 3014, 3015), and display(s) 3040 (e.g., 3010) can be used to control functions, and display information, related to the use of the device 3000. In some implementations, the controller can comprise an audio component that provides an audible indicator that identifies when the torque limit has been reached (e.g., or other alerts for the user).

The distal tip engagement portion 3004 can comprise housing 3046 that housed a non-powered distal tip shaft 3042, that is operably engaged with the engagement tip to provide rotational power to an engaged fastener, for example, during operation. Further, the distal tip engagement portion 3004 comprises a counter-torque engagement end 3024, that is configured to operably engage a target component or surface to provide counter-torque to the torque provided to the engagement tip 3008 by the tip shaft 3042. Some implementations, the distal tip engagement portion (e.g., or the engagement tip 3008, and/or the counter-torque engagement end 3024) can be selectably swapped with different version, with each version configured to engage with a corresponding target operation. That is, for example, a first fastener may utilize a first tip, while a second fastener may utilize a second tip. Additionally, a first counter-torque engagement end may engage with a first component or surface, while a second counter-torque engagement end may engage with a second component or surface. In this way, the appropriate tip portion 3004 can be selected for the corresponding operation. As an example, different distal tips can be designed for set screw tightening, drilling, tapping, counter-torque wrench operations, and any other rotary operation that utilizes torque and counter-torque.

In some implementations, the power source portion 3002 can be configured to be aseptically cleaned after use (e.g., after a surgical operation). The power source portion 3002 can be recharged (e.g., in a charging station), and reused. Further, the torque limiter 2300 can be aseptically cleaned after use, and reused, or may be disposable, with a new limiter used in a subsequent operation. Additionally, the distal tip engagement portion 3004 can be sterilized, such as by autoclaving, after being separated from the power source portion 3002; or the distal tip engagement portion 3004 may be disposable, and replaced after use.

In some implementations, after the torque limit has been reached, the binding force/torque between engagement tip 3008 and the target component (e.g., fastener) can be released with a very small, automatic counter rotation of the motor (e.g., less than 2 degrees), which does not affect the final applied torque. That is, for example, often, when applying torque to a component, the tool can be bound to the target component, which makes it difficult to remove the tool from the component without potentially adjusting the position of the component. In this implementation, in combination with the motor 3028, a clutch 3048 can be used to automatically apply a slight counter-rotation to the engagement tip 3008 to unbind the tool from the target component. In some implementations, the processor 3036 in the controller 3032 can be programmed to have the motor 3028 perform a small counter-rotation (e.gt., reverses the motor) when the forward rotation activation is released (e.g., deactivated, such as at the input switch 3038). In this way, merely enough counter-rotation can be applied to unbind the tool tip from the target component, and not undo any torque already applied to the target component.

In some implementations, the device 3000 can be configured to be used for a cannulation procedure. That is, cannulation typically introduces a wire, or the like, to be used as a guide for insertion of a component (e.g., bone screws) in pre-drilled holes. For example, a wire can be placed in a predrilled hole, and the wire can be run through a target screw, through the engagement tip 3008, the distal tip engagement portion 3004, and power source portion 3002. In this way, for example, the screw will be automatically aligned with the pre-drilled hole for improved placement. In this implementation, the engagement tip can comprise a central cavity to receive the wire, and respective shafts may also comprise a central cavity for receiving the wire. Additionally, in some implementations the motor 3028 can comprise a centrally-disposed cavity to receive the wire; or the motor 30287 can be disposed off-center in the housing 3044 (e.g., using off-set gearing to drive the shaft), to allow the wire to pass by the motor 3028.

The word "exemplary" is used herein to mean serving as an example, instance or illustration. Any aspect or design described herein as "exemplary" is not necessarily to be construed as advantageous over other aspects or designs. Rather, use of the word exemplary is intended to present concepts in a concrete fashion. As used in this application, the term "or" is intended to mean an inclusive "or" rather than an exclusive "or." That is, unless specified otherwise, or clear from context, "X employs A or B" is intended to mean any of the natural inclusive permutations. That is, if X employs A; X employs B; or X employs both A and B, then "X employs A or B" is satisfied under any of the foregoing instances. Further, at least one of A and B and/or the like generally means A or B or both A and B. In addition, the articles "a" and "an" as used in this application and the appended claims may generally be construed to mean "one or more" unless specified otherwise or clear from context to be directed to a singular form.

Although the subject matter has been described in language specific to structural features and/or methodological acts, it is to be understood that the subject matter defined in the appended claims is not necessarily limited to the specific features or acts described above. Rather, the specific features and acts described above are disclosed as example forms of implementing the claims.

Also, although the disclosure has been shown and described with respect to one or more implementations, equivalent alterations and modifications will occur to others skilled in the art based upon a reading and understanding of this specification and the annexed drawings. The disclosure includes all such modifications and alterations and is limited only by the scope of the following claims. In particular regard to the various functions performed by the above described components (e.g., elements, resources, etc.), the terms used to describe such components are intended to correspond, unless otherwise indicated, to any component which performs the specified function of the described component (e.g., that is functionally equivalent), even though not structurally equivalent to the disclosed structure which performs the function in the herein illustrated exemplary implementations of the disclosure. In addition, while a particular feature of the disclosure may have been disclosed with respect to only one of several implementations, such feature may be combined with one or more other features of the other implementations as may be desired and advantageous for any given or particular application. Furthermore, to the extent that the terms "includes," "having," "has," "with," or variants thereof are used in either the detailed description or the claims, such terms are intended to be inclusive in a manner similar to the term "comprising."

The implementations have been described, hereinabove. It will be apparent to those skilled in the art that the above methods and apparatuses may incorporate changes and modifications without departing from the general scope of this invention. It is intended to include all such modifications and alterations in so far as they come within the scope of the appended claims or the equivalents thereof.

Embodiments of the invention are set out in the following clauses:
1. A system comprising a device that applies torque to a target component that is engaged with a base component, and the device also applies counter-torque to the base component while the target component is rotated with regard to the base, the device comprising:
   a power source portion disposed at a proximal end;
   a tip engagement portion disposed at a distal end;
   a rotating shaft that rotates around a longitudinal axis relative to the tip engagement portion to operably provide torque to an engagement tip tool disposed at the distal end of the tip engagement portion, the engagement tip tool configured to selectably, fixedly engage the target component to provide rotation to the target component; and
   a counter-torque component comprising a body that is configured to selectably, fixedly engage with the base component to operably apply counter-torque to the base component.
2. The system of clause 1, comprising a torque limiter operably disposed between and engaged with the power source portion and the tip engagement portion, the torque limiter providing a pre-selected torque limit and operably mitigating further application of torque upon reaching the pre-selected torque limit.
3. The system of clause 2, the power source portion and the tip engagement portion selectably detachable from each other, and the torque limiter comprising a selectably replaceable unit.
4. The system of clause 1, comprising a plurality of torque limiters, respective torque limiters preset at a specified and different torque limit that operably mitigates further application of torque upon reaching the specified torque limit, and respective torque limiters selectably insertable between the power source portion and the tip engagement portion, thereby setting the specified torque limit for the device by selecting the corresponding torque limiter.
5. The system of clause 2, the torque limiter comprising:
   an input component comprising a body having plurality of radially spaced noses, the body fixedly engaged with a central power input coupler that is configured to operably engage a power output shaft of the power source portion of the device; and
   an output component comprising a wall having a plurality of inwardly projecting protrusions, wherein the number of protrusions matches the number of noses;
   wherein engagement of a nose of the plurality of noses with a protrusion of the plurality of protrusions transfers rotational power from the input component to the output component; and
   wherein the wall is configured to deflect outwardly to allow the engaged nose to rotate past the protrusion when the preset torque limit for the torque limiter is met.
6. The system of clause 5, the torque limiter comprising a leaf spring that is fixedly engaged with the central power input coupler and that radiates outward ending in a tip, wherein the thickened tip strikes the wall to generate an indication when the engaged nose rotates past the protrusion.
7. The system of clause 5, the protrusion formed by the wall gradually sloping inward in a direction of a first rotational action to a sharp shoulder that radiates outward to provide a first torque limit, wherein a direction of a second rotational action provides a second torque limit.
8. The system of clause 7, wherein the first torque limit is less than the second torque limit.
9. The system of clause 3, the torque limiter comprising:
   a sealed unit such that fluid leakage into the unit is mitigated, and that is configured to be cleaned on its exterior and reused; or
   an unsealed unit that is not configured to be cleaned and reused.
10. The system of clause 1, the tip engagement portion comprising:
   the rotating shaft that is selectably, operably engaged with:
      the engagement tip tool in a fixed engagement, wherein the engagement tip tool is configured to operably engage with, and apply torque to, the target component; and
      a power output shaft of the power source portion; and
   the counter-torque component.
11. The system of clause 10, the tip engagement portion comprising a selectably replaceable body, wherein a first selectably replaceable body comprises a first engagement tip tool configured to engage a first target component, and the first body comprises a first counter-torque component configured to engage a first target base component, and wherein a second selectably replaceable body comprises a second engagement tip tool configured to engage a second target component, and the second body comprises a second counter-torque component configured to engage a second target base component.
12. The system of clause 10, comprising:
   a plurality of selectably replaceable engagement tip tools, wherein a first selectably replaceable engagement tool tip is configured to engage a first target component, and a second selectably replaceable engagement tool tip is configured to engage a second target component; and
   a plurality of selectably replaceable counter-torque components, wherein a first selectably replaceable counter-torque component is configured to engage a first target base component, and a second selectably replaceable counter-torque component is configured to engage a second target base component.
13. The system of clause 1, the power source portion comprising:
   a rechargeable battery;
   a motor receiving electrical power from the battery;
   a controller that operably controls operation of the motor;
   a power output shaft that provides rotational power from the motor to the tip engagement portion; and
   a gearbox comprising gears that are configured to convert torque between the motor and the power output shaft.
14. The system of clause 13, the power source portion comprising an image sensor to collect image data of a target operation area and an illumination component to provide light to the target operation area.
15. The system of clause 1, the power source portion comprising:
   a motor receiving electrical power from an electrical power source;
   a controller that operably controls operation of the motor, the controller comprising:
      memory that stores data indicative of a pre-set torque limit; and
      a processor that identifies when an electrical current draw applied by the motor reaches a level indicative of the pre-set torque limit and shuts down the motor.
16. The system of clause 15, the controller storing data, in the memory and/or externally to the device, indicative of one or more of: time of device use, torque applied during use; torque limit during device use, torque profiles while tightening, and image data collected during use.
17. The system of clause 1, the power source portion comprising a motor and a controller that operably controls operation of the motor, wherein the controller reverses the motor upon deactivation of a forward activation of the motor, and wherein the reversal comprises merely enough to unbind the engagement tip tool from the target component.
18. The system of clause 1, the power source portion comprising a display that operably indicates to a user an amount of torque applied by the rotating shaft.
19. A device that applies torque to a target component that is engaged with a base component, and also applies counter-torque to the base component while the target component is rotated with regard to the base, the device comprising:
   a body comprising a proximal end and a distal end;
   a power source portion disposed at the proximal end;
   a tip engagement portion disposed at the distal end;
   a rotating shaft that rotates and provides torque around a longitudinal axis relative to the tip engagement portion;
   a torque limiter disposed between, and engaged with, the power source portion and the tip engagement portion, the torque limiter operable to provide a pre-selected torque limit and operably mitigate further application of torque by the rotating shaft upon reaching the pre-selected torque limit, wherein the torque limiter is selectably replaceable;
   an engaged engagement tip tool disposed at the distal end of the tip engagement portion, the engagement tip tool configured to selectably, fixedly engage with the target component to provide rotation to the target component; and
   a counter-torque component comprising a body that is configured to selectably, fixedly engage with the base component to operably apply counter-torque to the base component.
20. A device that applies torque to a target component that is engaged with a base component, and also applies counter-torque to the base component while the target component is rotated with regard to the base, the device comprising:
   a body comprising a proximal end and a distal end;
   a power source portion disposed at the proximal end;
   a tip engagement portion disposed at the distal end;
   a rotating shaft that rotates and provides torque around a longitudinal axis relative to the tip engagement portion;
   a torque limiter disposed between, and engaged with, the power source portion and the tip engagement portion, the torque limiter operable to provide a pre-selected torque limit and operably mitigate further application of torque by the rotating shaft upon reaching the pre-selected torque limit, wherein the torque limiter comprises:
      an input component comprising an input coupler that operably engages with, and receives rotational power from, an output of the power source portion, and a plurality of noses that project radially outward from the input coupler; and
      an output component comprising a wall having a plurality of projections that project radially inward to operably engage with the noses to transfer rotational power from the input component to the output component;
   an engaged engagement tip tool disposed at the distal end of the tip engagement portion, the engagement tip tool configured to selectably, fixedly engage with the target component to provide rotation to the target component; and
   a counter-torque component comprising a body that is configured to selectably, fixedly engage with the base component to operably apply counter-torque to the base component.

## Claims

1. A system comprising a device that applies torque to a target component that is engaged with a base component, and the device also applies counter-torque to the base component while the target component is rotated with regard to the base, the device comprising:
a power source portion disposed at a proximal end;
a tip engagement portion disposed at a distal end;
a rotating shaft that rotates around a longitudinal axis relative to the tip engagement portion to operably provide torque to an engagement tip tool disposed at the distal end of the tip engagement portion, the engagement tip tool configured to selectably, fixedly engage the target component to provide rotation to the target component; and
a counter-torque component comprising a body that is configured to selectably, fixedly engage with the base component to operably apply counter-torque to the base component.

2. The system of claim 1, comprising a torque limiter operably disposed between and engaged with the power source portion and the tip engagement portion, the torque limiter providing a pre-selected torque limit and operably mitigating further application of torque upon reaching the pre-selected torque limit.

3. The system of claim 2, the power source portion and the tip engagement portion selectably detachable from each other, and the torque limiter comprising a selectably replaceable unit.

4. The system of any preceding claim, comprising a plurality of torque limiters, respective torque limiters preset at a specified and different torque limit that operably mitigates further application of torque upon reaching the specified torque limit, and respective torque limiters selectably insertable between the power source portion and the tip engagement portion, thereby setting the specified torque limit for the device by selecting the corresponding torque limiter.

5. The system of claim 2, the torque limiter comprising:
an input component comprising a body having plurality of radially spaced noses, the body fixedly engaged with a central power input coupler that is configured to operably engage a power output shaft of the power source portion of the device; and
an output component comprising a wall having a plurality of inwardly projecting protrusions, wherein the number of protrusions matches the number of noses;
wherein engagement of a nose of the plurality of noses with a protrusion of the plurality of protrusions transfers rotational power from the input component to the output component; and
wherein the wall is configured to deflect outwardly to allow the engaged nose to rotate past the protrusion when the preset torque limit for the torque limiter is met.

6. The system of claim 5, the torque limiter comprising a leaf spring that is fixedly engaged with the central power input coupler and that radiates outward ending in a tip, wherein the thickened tip strikes the wall to generate an indication when the engaged nose rotates past the protrusion, and/or
wherein the protrusion is formed by the wall gradually sloping inward in a direction of a first rotational action to a sharp shoulder that radiates outward to provide a first torque limit, wherein a direction of a second rotational action provides a second torque limit, preferably wherein the first torque limit is less than the second torque limit.

7. The system of claim 3, the torque limiter comprising:
a sealed unit such that fluid leakage into the unit is mitigated, and that is configured to be cleaned on its exterior and reused; or
an unsealed unit that is not configured to be cleaned and reused.

8. The system of any preceding claim, the tip engagement portion comprising:
the rotating shaft that is selectably, operably engaged with:
the engagement tip tool in a fixed engagement, wherein the engagement tip tool is configured to operably engage with, and apply torque to, the target component; and
a power output shaft of the power source portion; and
the counter-torque component.

9. The system of claim 8, the tip engagement portion comprising a selectably replaceable body, wherein a first selectably replaceable body comprises a first engagement tip tool configured to engage a first target component, and the first body comprises a first counter-torque component configured to engage a first target base component, and wherein a second selectably replaceable body comprises a second engagement tip tool configured to engage a second target component, and the second body comprises a second counter-torque component configured to engage a second target base component.

10. The system of claim 8, comprising:
a plurality of selectably replaceable engagement tip tools, wherein a first selectably replaceable engagement tool tip is configured to engage a first target component, and a second selectably replaceable engagement tool tip is configured to engage a second target component; and
a plurality of selectably replaceable counter-torque components, wherein a first selectably replaceable counter-torque component is configured to engage a first target base component, and a second selectably replaceable counter-torque component is configured to engage a second target base component.

11. The system of any preceding claim, the power source portion comprising:
a rechargeable battery;
a motor receiving electrical power from the battery;
a controller that operably controls operation of the motor;
a power output shaft that provides rotational power from the motor to the tip engagement portion; and a gearbox comprising gears that are configured to convert torque between the motor and the power output shaft, preferably wherein the power source portion comprises an image sensor to collect image data of a target operation area and an illumination component to provide light to the target operation area.

12. The system of any one of claims 1 to 10, wherein the power source portion comprises:
a motor receiving electrical power from an electrical power source;
a controller that operably controls operation of the motor, the controller comprising:
memory that stores data indicative of a pre-set torque limit; and
a processor that identifies when an electrical current draw applied by the motor reaches a level indicative of the pre-set torque limit and shuts down the motor, preferably wherein the controller stores data, in the memory and/or externally to the device, indicative of one or more of: time of device use, torque applied during use; torque limit during device use, torque profiles while tightening, and image data collected during use.

13. The system of any one of claims 1 to 10, wherein the power source portion comprises a motor and a controller that operably controls operation of the motor, wherein the controller reverses the motor upon deactivation of a forward activation of the motor, and wherein the reversal comprises merely enough to unbind the engagement tip tool from the target component, and/or wherein the power source portion comprising a display that operably indicates to a user an amount of torque applied by the rotating shaft.

14. A device that applies torque to a target component that is engaged with a base component, and also applies counter-torque to the base component while the target component is rotated with regard to the base, the device comprising:
a body comprising a proximal end and a distal end;
a power source portion disposed at the proximal end;
a tip engagement portion disposed at the distal end;
a rotating shaft that rotates and provides torque around a longitudinal axis relative to the tip engagement portion;
a torque limiter disposed between, and engaged with, the power source portion and the tip engagement portion, the torque limiter operable to provide a pre-selected torque limit and operably mitigate further application of torque by the rotating shaft upon reaching the pre-selected torque limit, wherein the torque limiter is selectably replaceable;
an engaged engagement tip tool disposed at the distal end of the tip engagement portion, the engagement tip tool configured to selectably, fixedly engage with the target component to provide rotation to the target component; and
a counter-torque component comprising a body that is configured to selectably, fixedly engage with the base component to operably apply counter-torque to the base component.

15. A device that applies torque to a target component that is engaged with a base component, and also applies counter-torque to the base component while the target component is rotated with regard to the base, the device comprising:
a body comprising a proximal end and a distal end;
a power source portion disposed at the proximal end;
a tip engagement portion disposed at the distal end;
a rotating shaft that rotates and provides torque around a longitudinal axis relative to the tip engagement portion;
a torque limiter disposed between, and engaged with, the power source portion and the tip engagement portion, the torque limiter operable to provide a pre-selected torque limit and operably mitigate further application of torque by the rotating shaft upon reaching the pre-selected torque limit, wherein the torque limiter comprises:
an input component comprising an input coupler that operably engages with, and receives rotational power from, an output of the power source portion, and a plurality of noses that project radially outward from the input coupler; and
an output component comprising a wall having a plurality of projections that project radially inward to operably engage with the noses to transfer rotational power from the input component to the output component;
an engaged engagement tip tool disposed at the distal end of the tip engagement portion, the engagement tip tool configured to selectably, fixedly engage with the target component to provide rotation to the target component; and
a counter-torque component comprising a body that is configured to selectably, fixedly engage with the base component to operably apply counter-torque to the base component.
